# EUROPÄISCHE PATENTSCHRIFT

(11) **EP 2 931 698 B1**
(45) Veröffentlichungstag und Bekanntmachung des Hinweises auf die Patenterteilung: **13.09.2017**
(21) Anmeldenummer: 13792263.9
(22) Anmeldetag: 15.11.2013
(51) Int. Cl.: C07D 405/14, C07D 403/04, C07D 403/14, C09K 11/02, C09K 11/06, C08G 73/06, H01L 51/00, H01L 51/50

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 14.12.2012 EP 12008332
(43) Veröffentlichungstag der Anmeldung: 21.10.2015
(73) Patentinhaber: Merck Patent GmbH, 64293 Darmstadt (DE)
(72) Erfinder: PARHAM, Amir Hossain, 60486 Frankfurt am Main (DE); EBERLE, Thomas, 76829 Landau (DE); JATSCH, Anja, 60489 Frankfurt am Main (DE); PFLUMM, Christof, 64291 Darmstadt (DE); KROEBER, Jonas Valentin, 60311 Frankfurt am Main (DE); ANEMIAN, Rémi Manouk, Seoul 657-169 (KR)
(86) Internationale Anmeldenummer: PCT/EP2013/003456
(87) Internationale Veröffentlichungsnummer: WO 2014/090368

(56) Entgegenhaltungen:
- DE-A1-102009 023 155
- KR-A- 20120 072 787

## Beschreibung

Die vorliegende Anmeldung betrifft eine Verbindung einer Formel (I), welche eine Indenocarbazol-, eine Carbazol- und eine elektronenarme Heteroarylgruppe in einem Molekül aufweist. Die Verbindung kann in einer elektronischen Vorrichtung verwendet werden.

Unter elektronischen Vorrichtungen im Sinne dieser Anmeldung werden insbesondere sogenannte organische elektronische Vorrichtungen verstanden (organic electronic devices), welche organische Halbleitermaterialien als Funktionsmaterialien enthalten. Nochmals insbesondere werden darunter organische Elektrolumineszenzvorrichtungen (OLEDs) und andere elektronische Vorrichtungen verstanden, welche im Folgenden bei der detaillierten Beschreibung der Erfindung aufgeführt sind.

Allgemein wird unter der Bezeichnung OLED eine elektronische Vorrichtung verstanden, welche mindestens ein organisches Material enthält und unter Anlegen einer elektrischen Spannung Licht emittiert. Der genaue Aufbau von OLEDs ist unter anderem in US 4539507, US 5151629, EP 0676461 und WO 98/27136 beschrieben.

Bei elektronischen Vorrichtungen, insbesondere OLEDs, besteht großes Interesse an der Verbesserung der Leistungsdaten, insbesondere Lebensdauer und Effizienz und Betriebsspannung. Eine wichtige Rolle spielen dabei organische Emitterschichten, insbesondere die darin enthaltenen Matrixmaterialien, und organische Schichten mit elektronentransportierender Funktion.

Zur Lösung dieser technischen Aufgabe werden kontinuierlich neue Materialien gesucht, die sich zur Verwendung als Matrixmaterialien in emittierenden Schichten, insbesondere phosphoreszierenden emittierenden Schichten, eignen. Weiterhin werden Materialien mit elektronentransportierenden und/oder lochblockierenden Eigenschaften zur Verwendung in entsprechenden Funktionsschichten gesucht.

Phosphoreszierende emittierende Schichten im Sinne der vorliegenden Anmeldung sind solche organischen Schichten, welche mindestens eine phosphoreszierende Emitterverbindung enthalten.

Emitterverbindungen einer emittierenden Schicht sind typischerweise Verbindungen, welche bei Betrieb der elektronischen Vorrichtung Licht emittieren.

Vom Begriff phosphoreszierende Emitter sind gemäß der vorliegenden Anmeldung Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, wie einem Quintett-Zustand.

Unter einem Matrixmaterial wird in einem System enthaltend zwei oder mehr Materialien diejenige Komponente verstanden, deren Anteil in der Mischung der größere ist. Entsprechend wird unter einem Dotanden in einem System enthaltend zwei oder mehr Materialien diejenige Komponente verstanden, deren Anteil in der Mischung der kleinere ist. Statt des Begriffs Matrixmaterial wird in vielen Fällen auch der Begriff Hostmaterial verwendet.

Wird eine Emitterverbindung in Kombination mit einer oder mehreren weiteren Verbindungen in einer emittierenden Schicht verwendet, so ist ihr Anteil in der Mischung typischerweise der relativ kleinere. Sie kann in diesem Fall auch als Dotandverbindung bezeichnet werden. Die eine oder mehreren weiteren Verbindungen sind typischerweise in relativ größerem Anteil in der Mischung vorhanden und können daher gemäß der obenstehenden Definition als Matrixmaterialien bezeichnet werden.

Im Stand der Technik bekannt ist die Verwendung von Verbindungen enthaltend eine oder mehrere Carbazolgruppen in elektronischen Vorrichtungen, beispielsweise aus WO 2005/039246, US 2005/0069729, JP 2004/288381, EP 1205527 oder WO 2008/086851.

Weiterhin im Stand der Technik bekannt ist die Verwendung von Verbindungen enthaltend eine oder mehrere Indenocarbazolgruppen in elektronischen Vorrichtungen, beispielsweise aus DE-A-10 2009 023155, WO 2010/136109 und WO 2011/000455.
Weiterhin im Stand der Technik bekannt ist die Verwendung von Verbindungen enthaltend eine oder mehrere elektronenarme heteroaromatische Sechsringe in elektronischen Vorrichtungen, beispielsweise aus WO 2010/015306, WO 2007/063754 und WO 2008/056746.

Es besteht jedoch weiterhin Bedarf an alternativen Verbindungen zur Verwendung in elektronischen Vorrichtungen, insbesondere zur Verwendung als Matrixmaterialien für phosphoreszierende emittierende Schichten von organischen Elektrolumineszenzvorrichtungen.

Nochmals weiterhin ist es im Stand der Technik bekannt, Verbindungen in elektronischen Vorrichtungen einzusetzen, welche sowohl einen oder mehrere elektronenarme heteroaromatische Sechsringe, eine oder mehrere Carbazolgruppen und eine oder mehrere Indenocarbazolgruppen aufweisen. Solche Verbindungen und ihre Verwendung in elektronischen Vorrichtungen sind beispielsweise in WO 2010/136109, WO 2011/000455, WO 2012/069121 und WO 2012/014500 offenbart.
Es besteht jedoch weiterhin Verbesserungsbedarf gegenüber diesen Verbindungen, insbesondere in den Punkten Betriebsspannung, Leistungseffizienz und Lebensdauer von Vorrichtungen enthaltend die Verbindungen.
Überraschend wurde nun gefunden, dass mit einer bestimmten isomeren Indenocarbazol-Verbindung, die über ihr N-Atom mit einem elektronenarmen Sechsring-Heteroaromaten verbunden ist, und die einen Carbazolsubstituenten über eines seiner Kohlenstoffatom gebunden trägt, hervorragende Leistungsdaten erzielt werden können. Insbesondere werden hervorragende Lebensdauer und Leistungseffizienz bei Verwendung in organischen Elektrolumineszenzvorrichtungen erreicht.

Gegenstand der vorliegenden Anmeldung ist somit eine Verbindung einer Formel (I)

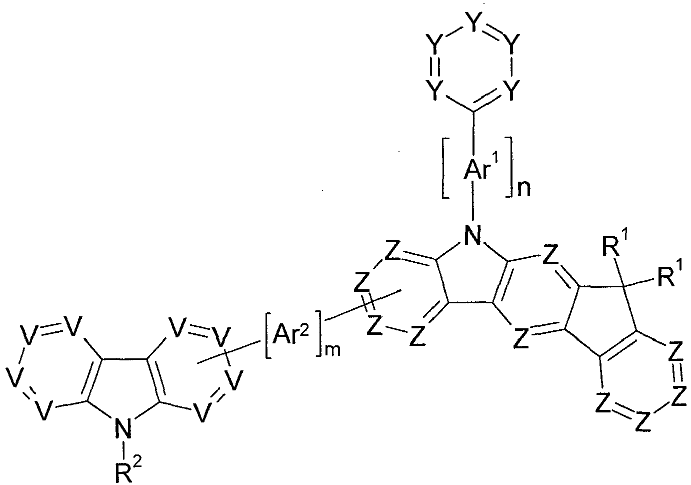

Formel (I), wobei gilt:
- Y: ist gleich N oder CR¹, wobei mindestens zwei Gruppen Y im Sechsring gleich N sein müssen;
- Z: ist gleich CR¹ oder N;
- V: ist gleich CR² oder N;
- Ar¹: ist ein aromatisches oder heteroaromatisches Ringsystem mit 6 bis 40 aromatischen Ringatomen, das mit einem oder mehreren Resten R¹ substituiert sein kann;
- Ar²: ist ein aromatisches oder heteroaromatisches Ringsystem mit 6 bis 40 aromatischen Ringatomen, das mit einem oder mehreren Resten R¹ substituiert sein kann;
- R¹: ist bei jedem Auftreten gleich oder verschieden H, D, F, Br, Cl, I, C(=O)R³, CN, Si(R³)₃, N(R³)₂, P(=O)(R³)₂, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch - R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann, wobei zwei oder mehr Reste R¹ miteinander verknüpft sein können und einen Ring bilden können;
- R²: ist bei jedem Auftreten gleich oder verschieden H, D, F, Br, Cl, I, C(=O)R³, CN, Si(R³)₃, N(R³)₂, P(=O)(R³)₂, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch - R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann;
- R³: ist bei jedem Auftreten gleich oder verschieden H, D, F, Br, Cl, I, C(=O)R⁴, CN, Si(R⁴)₃, N(R⁴)₂, P(=O)(R⁴)₂, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch - R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxy- oder Heteroaryloxygruppe mit 5 bis 30 aromatischen Ringatomen, die durch einen oder mehrere Reste R⁴ substituiert sein kann, wobei zwei oder mehr Reste R³ miteinander verknüpft sein können und einen Ring bilden können;
- R⁴: ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden;
- n: ist gleich 0 oder 1;
- m: ist gleich 0 oder 1;
wobei der Index n gleich 0 ist, wenn in Formel (I) sowohl
a) nicht mehr als zwei Gruppen Y vorliegen, die gleich N sind, als auch
b) sich diese zwei Gruppen Y, die gleich N sind, in meta-Position zueinander am Sechsring befinden.

Unter der Bezeichnung, dass sich Gruppen Y, die gleich N sind, in meta-Position zueinander am Sechsring befinden, wird im Rahmen der vorliegenden Anmeldung verstanden, dass die Gruppen Y, die gleich N sind, im Sechsring nicht benachbart sind, sondern dass genau eine andere Gruppe Y zwischen ihnen liegt. Die Gruppen Y, die gleich N sind, sind damit so im Sechsring angeordnet, dass die jeweils übernächste Gruppe Y diejenige Gruppe ist, die gleich N ist, und die nächste Gruppe Y gleich CR¹ ist.

Es gelten im Rahmen der vorliegenden Anmeldungen folgende Definitionen für chemische Gruppen:

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

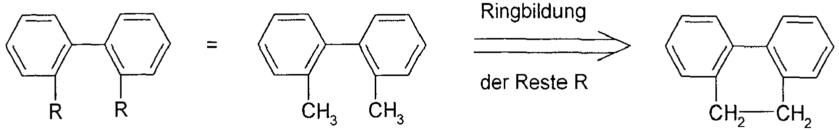

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

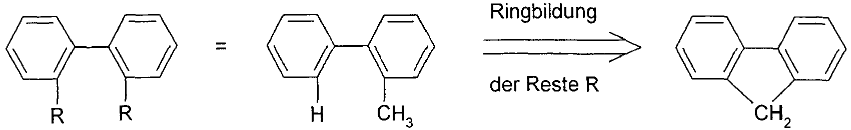

Gemäß einer bevorzugten Ausführungsform ist der Index m gleich 0.

Gemäß einer weiteren bevorzugten Ausführungsform ist der Index n gleich 0, wenn nicht mehr als zwei Gruppen Y im Sechsring gleich N sind. Gemäß einer besonders bevorzugten Ausführungsform ist der Index n gleich 0 oder 1, wenn genau drei Gruppen Y im Sechsring gleich N sind, und der Index n ist in allen anderen Fällen gleich 0.

Gemäß einer weiteren bevorzugten Ausführungsform sind höchstens drei Gruppen Z in einem aromatischen Ring gleich N, besonders bevorzugt höchstens zwei Gruppen Z in einem aromatischen Ring gleich N, und ganz besonders bevorzugt höchstens eine Gruppe Z in einem aromatischen Ring gleich N.

Weiterhin ist es bevorzugt, dass nicht mehr als zwei benachbarte Gruppen Z in einem Sechsring gleich N sind.

Es ist insbesondere bevorzugt, dass Z gleich CR¹ ist.

Gemäß einer weiteren bevorzugten Ausführungsform sind höchstens drei Gruppen V in einem aromatischen Ring gleich N, besonders bevorzugt höchstens zwei Gruppen V in einem aromatischen Ring gleich N, und ganz besonders bevorzugt höchstens eine Gruppe V in einem aromatischen Ring gleich N.

Weiterhin ist es bevorzugt, dass nicht mehr als zwei benachbarte Gruppen V in einem Sechsring gleich N sind.

Es ist insbesondere bevorzugt, dass V gleich CR² ist.

Es ist für die Gruppe Y bevorzugt, dass genau zwei oder genau drei Gruppen Y im Ring gleich N sind, und die restlichen Gruppen Y gleich CR¹ sind. Es ist besonders bevorzugt, dass genau drei Gruppen Y im Ring gleich N sind, und die restlichen Gruppen Y gleich CR¹ sind. Weiterhin ist für die Gruppe Y bevorzugt, dass nicht mehr als zwei benachbarte Gruppen Y gleich N sind, besonders bevorzugt, dass keine benachbarten Gruppen Y gleich N sind.

Gemäß einer weiteren bevorzugten Ausführungsform bilden Reste R¹ in Gruppen Y, die CR¹ darstellen, einen Ring miteinander. Bevorzugt sind dies Reste R¹ in benachbarten Gruppen Y, welche CR¹ darstellen. Besonders bevorzugt bilden in diesem Fall die Reste R¹ in benachbarten Gruppen Y, welche CR¹ darstellen, einen ankondensierten Benzolring. Ganz besonders bevorzugt sind in diesem Fall genau zwei Gruppen Y gleich N.

Für die Gruppe Ar¹ ist bevorzugt, dass sie eine Gruppe der folgenden Formel (Ar¹-I) darstellt 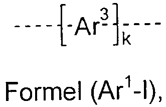 wobei die gestrichelten Linien die Bindungen an die Indenocarbazolgruppe und den Sechsring enthaltend die Gruppen Y darstellen,
- Ar³: bei jedem Auftreten gleich oder verschieden eine Aryl- oder Heteroarylgruppe mit 6 bis 18 aromatischen Ringatomen ist, die mit einem oder mehreren Resten R¹ substituiert sein kann, wobei R¹ definiert ist wie oben; und
- k: 1, 2, 3 oder 4 ist, wobei der Index k so gewählt ist, dass die Zahl der aromatischen Ringatome der gesamten Gruppe Ar¹ die Zahl 40 nicht überschreitet.

Ar³ ist bevorzugt bei jedem Auftreten gleich oder verschieden eine Aryl- oder Heteroarylgruppe mit 6 bis 14 aromatischen Ringatomen, besonders bevorzugt eine Aryl- oder Heteroarylgruppe mit 6 bis 10 aromatischen Ringatomen, und ganz besonders bevorzugt eine Aryl- oder Heteroarylgruppe mit 6 aromatischen Ringatomen, wobei die genannten Gruppen mit einem oder mehreren Resten R¹ substituiert sein können.

Gemäß einer bevorzugten Ausführungsform bilden Reste R¹ dabei Ringe zwischen den Aryl- oder Heteroarylgruppen Ar³, an die sie binden. Insbesondere bevorzugt werden dabei zwei Gruppen Ar³, die Phenyl darstellen, zu einer Fluorenylgruppe verbunden.

Der Index k ist bevorzugt 1, 2 oder 3, besonders bevorzugt 1 oder 2.

Bevorzugte Ausführungsformen der Gruppe Ar¹ entsprechen den im Folgenden angegebenen Formeln (Ar¹-I-1) bis (Ar¹-I-26)

| | |
|---|---|
| 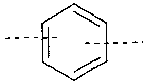 | 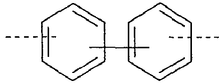 |
| Formel (Ar¹-I-1) | Formel (Ar¹-I-2) |
| 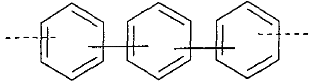 | 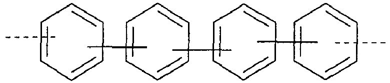 |
| Formel (Ar¹-I-3) | Formel (Ar¹-I-4) |
| 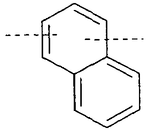 | 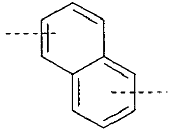 |
| Formel (Ar¹-I-5) | Formel (Ar¹-I-6) |
| 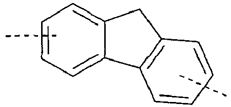 | 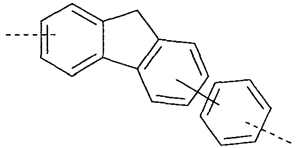 |
| Formel (Ar¹-I-7) | Formel (Ar¹-I-8) |
| 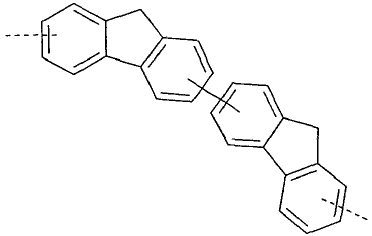 | 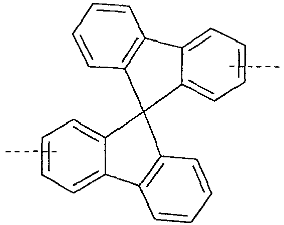 |
| Formel (Ar¹-I-9) | Formel (Ar¹-I-10) |
| 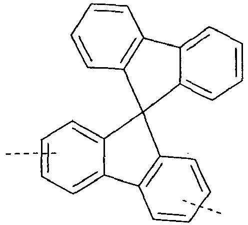 | 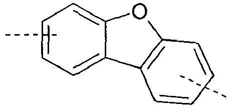 |
| Formel (Ar¹-I-11) | Formel (Ar¹-I-12) |
| 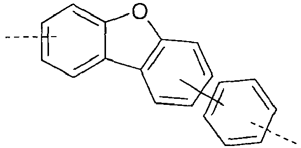 | 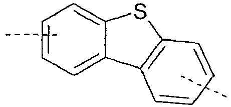 |
| Formel (Ar¹-I-13) | Formel (Ar¹-I-14) |
| 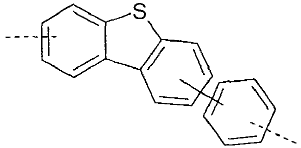 | 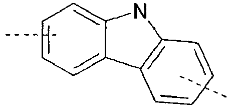 |
| Formel (Ar¹-I-15) | Formel (Ar¹-I-16) |
| 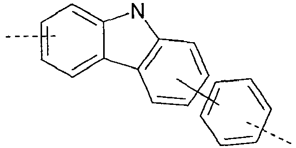 | 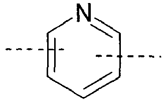 |
| Formel (Ar¹-I-17) | Formel (Ar¹-I-18) |
| 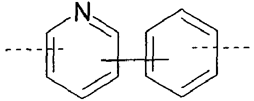 | 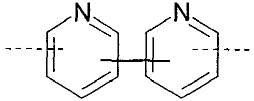 |
| Formel (Ar¹-I-19) | Formel (Ar¹-I-20) |
| 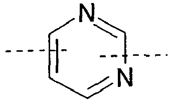 | 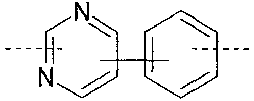 |
| Formel (Ar¹-I-21) | Formel (Ar¹-I-22) |
| 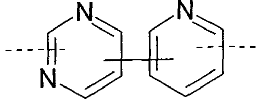 | 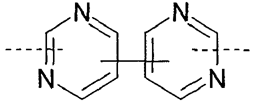 |
| Formel (Ar¹-I-23) | Formel (Ar¹-I-24) |
| 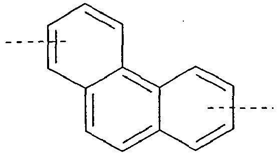 | 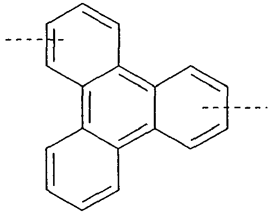 |
| Formel (Ar¹-I-25) | Formel (Ar¹-I-26) |

wobei die gestrichelten Linien die Bindungen an die Indenocarbazolgruppe und den Sechsring enthaltend die Gruppen Y darstellen,
und wobei die Gruppen an allen freien Positionen mit Resten R¹ substituiert sein können.

Für die Gruppe Ar² ist bevorzugt, dass sie gewählt ist aus einem aromatischen oder heteroaromatischen Ringsystem mit 6 bis 30 aromatischen Ringatomen, besonders bevorzugt 6 bis 24 aromatischen Ringatomen, das mit einem oder mehreren Resten R¹ substituiert sein kann.

Bevorzugte Ausführungsformen der Gruppe der Formel (Y) 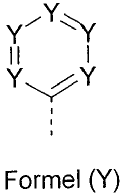 als Bestandteil der Formel (I) entsprechen den folgenden Formeln (Y-1) bis (Y-6)

| | |
|---|---|
| 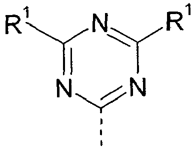 | 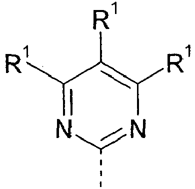 |
| Formel (Y-1) | Formel (Y-2) |
| 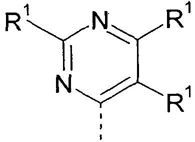 | 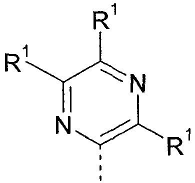 |
| Formel (Y-3) | Formel (Y-4) |
| 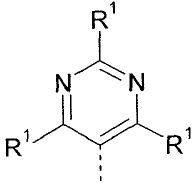 | 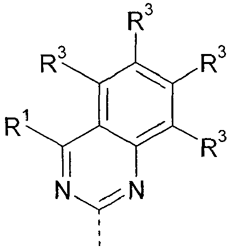 |
| Formel (Y-5) | Formel (Y-6) |

wobei die gestrichelte Linie die Bindung an den Rest der Verbindung bezeichnet und wobei R¹ und R³ wie oben definiert ist.

Besonders bevorzugt ist darunter Formel (Y-1).

Bevorzugt ist der Rest R¹ bei jedem Auftreten gleich oder verschieden H, D, F, CN, Si(R³)₃, N(R³)₂, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R³ substituiert sein können und wobei in den oben genannten Gruppen eine oder mehrere CH₂-Gruppen durch - C≡C-, -R³C=CR³-, Si(R³)₂, C=O, C=NR³, -NR³-, -O-, -S-, -C(=O)O- oder-C(=O)NR³- ersetzt sein können, oder ein aromatisches oder heteroaromatisches Ringsystem mit 5 bis 20 aromatischen Ringatomen, das jeweils mit einem oder mehreren Resten R³ substituiert sein kann, wobei zwei oder mehr Reste R¹ miteinander verknüpft sein können und einen Ring bilden können.

Bevorzugt ist der Rest R² bei jedem Auftreten gleich oder verschieden H, D, F, CN, Si(R³)₃, N(R³)₂, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R³ substituiert sein können und wobei in den oben genannten Gruppen eine oder mehrere CH₂-Gruppen durch-C≡C-, -R³C=CR³-, Si(R³)₂, C=O, C=NR³, -NR³-, -O-, -S-, -C(=O)O- oder-C(=O)NR³- ersetzt sein können, oder ein aromatisches oder heteroaromatisches Ringsystem mit 5 bis 20 aromatischen Ringatomen, das jeweils mit einem oder mehreren Resten R³ substituiert sein kann.

Bevorzugt ist der Rest R³ bei jedem Auftreten gleich oder verschieden H, D, F, CN, Si(R⁴)₃, N(R⁴)₂, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R⁴ substituiert sein können und wobei in den oben genannten Gruppen eine oder mehrere CH₂-Gruppen durch - C≡C-, -R⁴C=CR⁴-, Si(R⁴)₂, C=O, C=NR⁴, -NR⁴-, -O-, -S-, -C(=O)O- oder-C(=O)NR⁴- ersetzt sein können, oder ein aromatisches oder heteroaromatisches Ringsystem mit 5 bis 20 aromatischen Ringatomen, das jeweils mit einem oder mehreren Resten R⁴ substituiert sein kann, wobei zwei oder mehr Reste R³ miteinander verknüpft sein können und einen Ring bilden können.

Bezüglich der Bindung der Gruppe Ar² an das Indenocarbazol ist es bevorzugt, dass diese in folgender Formel (I-A) in den mit "a" und "b" bezeichneten Positionen vorliegt, besonders bevorzugt in der mit "a" bezeichneten Position. Entsprechendes gilt, wenn der Index m gleich 0 ist, für die anstelle von Ar² bindende Carbazolgruppe.

In der Formel (I-A) 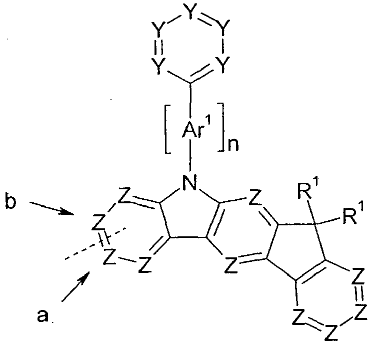 bezeichnet die gestrichelte Linie die Bindung an den Rest der Formel (I), und die oben genannten Positionen "a" und "b" sind mit Pfeilen gekennzeichnet.

Bezüglich der Bindung der Gruppe Ar² an das Carbazol ist es bevorzugt, dass diese in folgender Formel (I-B) in den mit "c" und "d" bezeichneten Positionen vorliegt, besonders bevorzugt in der mit "c" bezeichneten Position. Entsprechendes gilt, wenn der Index m gleich 0 ist, für die anstelle von Ar² bindende Indenocarbazolgruppe.

In der Formel (I-B) 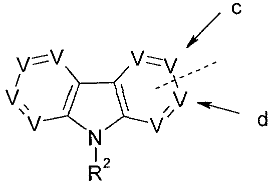 bezeichnet die gestrichelte Linie die Bindung an den Rest der Formel (I), und die oben genannten Positionen "c" und "d" sind mit Pfeilen bezeichnet.

Bevorzugte Ausführungsformen der Verbindung der Formel (I) entsprechen einer der folgenden Formeln (I-1) und (I-2) 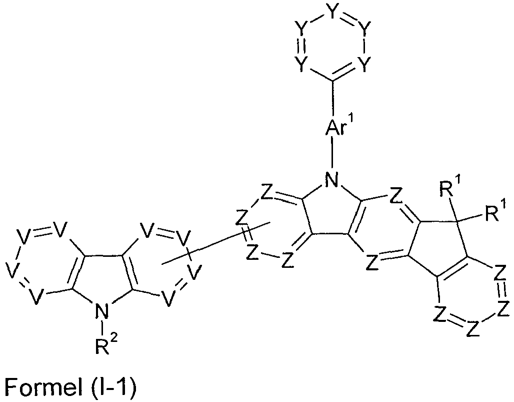 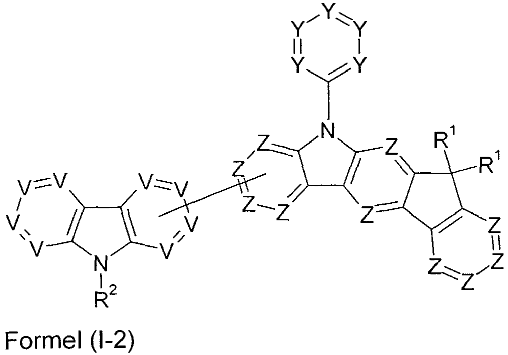 wobei die auftretenden Symbole wie oben definiert sind und für Formel (I-1) nicht der Fall auftreten darf, dass nicht mehr als zwei Gruppen Y vorliegen, die gleich N sind, und sich diese zwei Gruppen Y, die gleich N sind, in meta-Position zueinander am Sechsring befinden.

Bevorzugt sind für Formel (I-1) mehr als zwei, besonders bevorzugt genau drei Gruppen Y im Sechsring gleich N.

Weiterhin gelten für die Formeln (I-1) und (I-2) bevorzugt die oben angegebenen bevorzugten Ausführungsformen der Gruppen Ar¹, Z, V, R¹ und R².

Weiterhin gelten die oben angegebenen bevorzugten Ausführungsformen betreffend die Position der Bindung zwischen Indenocarbazol und Carbazol ebenfalls als bevorzugt.

Weiterhin bevorzugt entspricht der Sechsring enthaltend die Gruppen Y in den Formeln (I-1) und (I-2) bevorzugt einer der oben angegebenen Formeln (Y-1) bis (Y-6), besonders bevorzugt der Formel (Y-1).

Besonders bevorzugte Ausführungsformen für Verbindungen der Formel (I) entsprechen den folgenden Formeln (I-1-1) bis (I-1-3) und (I-2-1) bis (I-2-3) 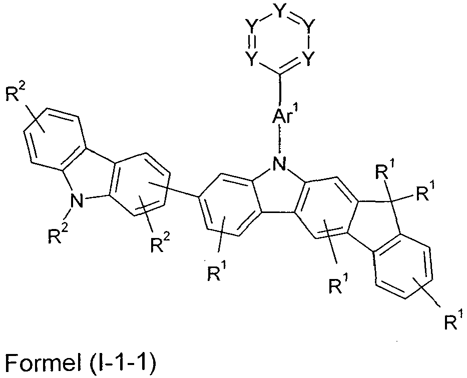 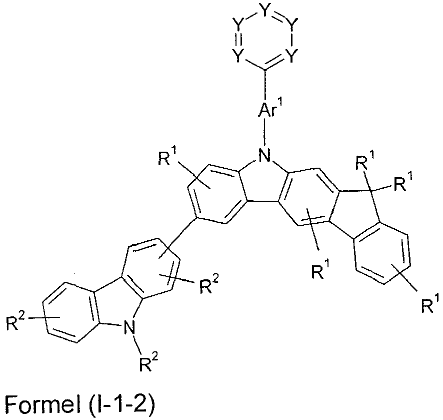 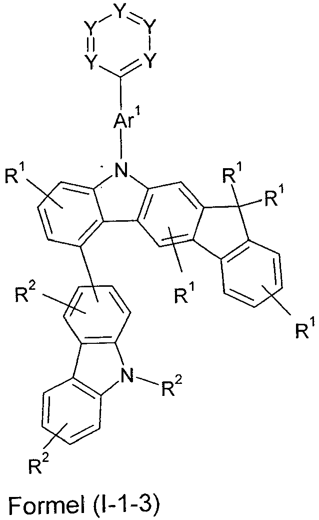 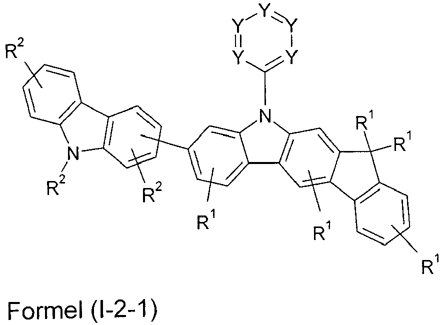 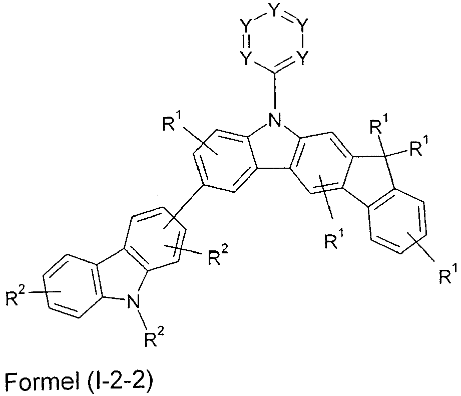 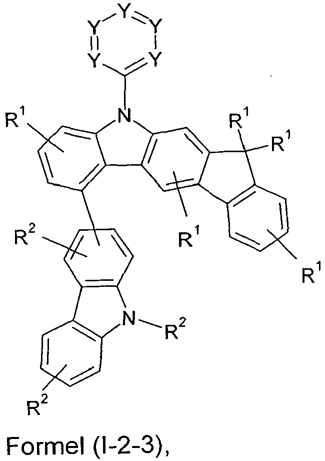 wobei die auftretenden Gruppen wie oben definiert sind,
und wobei ein gebundener Rest R¹ bzw. R² an einem Sechsring bedeutet, dass alle freien Positionen des Sechsrings jeweils mit Resten R¹ bzw. R² substituiert sein können,
und wobei für die Formeln (I-1-1) bis (I-1-3) mehr als zwei, bevorzugt genau drei Gruppen Y im Sechsring gleich N sind.

Weiterhin gelten für die Formeln (I-1-1) bis (I-1-3) und (I-2-1) bis (I-2-3) bevorzugt die oben angegebenen bevorzugten Ausführungsformen der Gruppen Ar¹, R¹ und R².

Weiterhin ist es bevorzugt, dass das Carbazol in den oben genannten Formeln in einer der Positionen "c" und "d" gebunden ist, wie oben für Formel (I-B) angegeben, besonders bevorzugt in Position "c".

Weiterhin bevorzugt entspricht der Sechsring enthaltend die Gruppen Y in den oben genannten Formeln bevorzugt einer der oben angegebenen Formeln (Y-1) bis (Y-6), besonders bevorzugt der Formel (Y-1).

Besonders bevorzugt sind unter den Formeln (I-1-1) bis (I-1-3) und (I-2-1) bis (I-2-3) die beiden Formeln (I-1-2) und (I-2-2), insbesondere in Kombination mit den oben angegebenen bevorzugten Ausführungsformen der Gruppen R¹ und R² und Ar¹.

Die folgende Tabelle zeigt Beispiele für Verbindungen gemäß Formel (I):

| | |
|---|---|
| 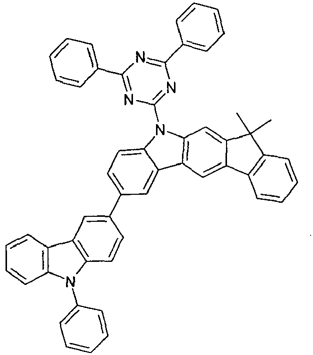 | 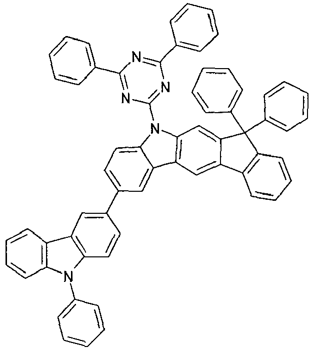 |
| 1 | 2 |
| 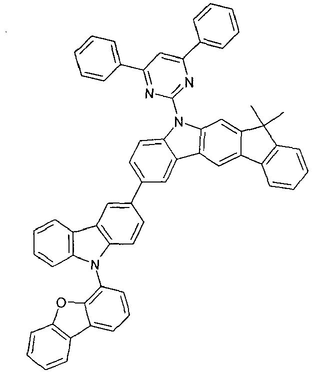 | 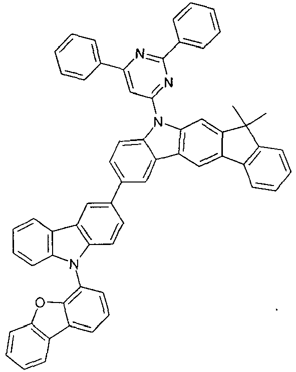 |
| 3 | 4 |
| 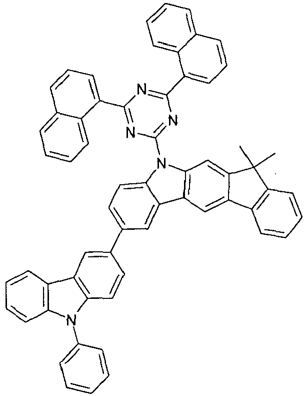 | 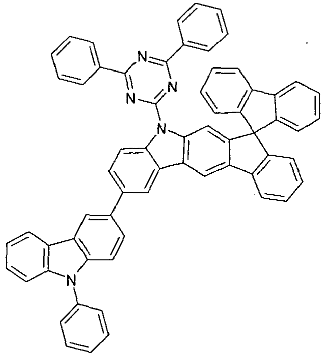 |
| 5 | 6 |
| 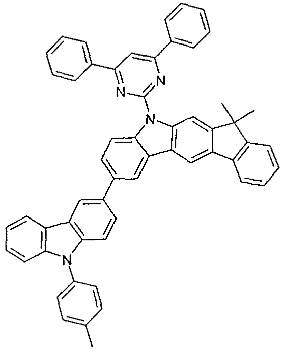 | 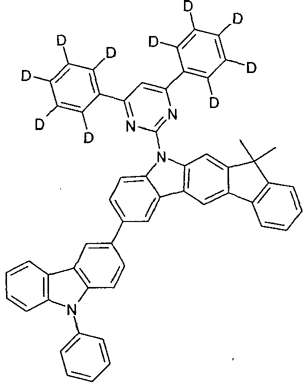 |
| 7 | 8 |
| 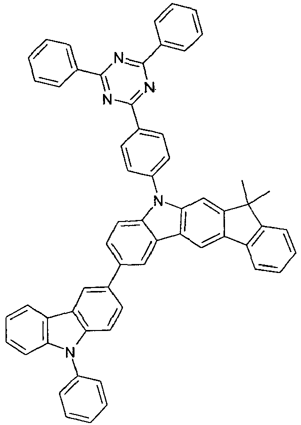 | 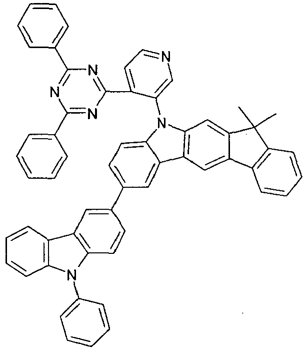 |
| 9 | 10 |
| 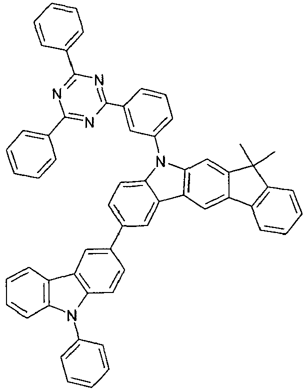 | 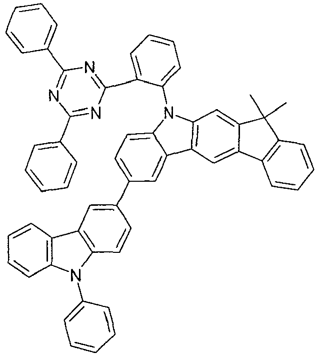 |
| 11 | 12 |
| 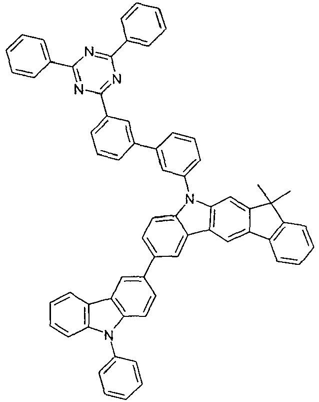 | 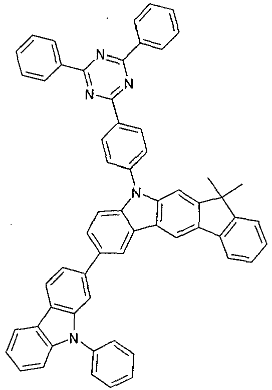 |
| 13 | 14 |
| 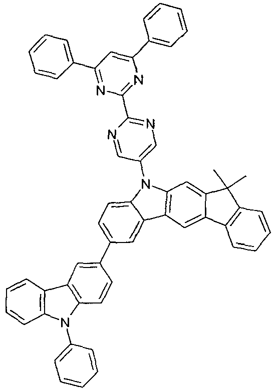 | 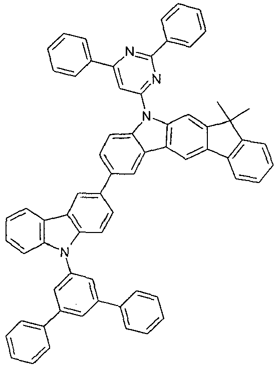 |
| 15 | 16 |
| 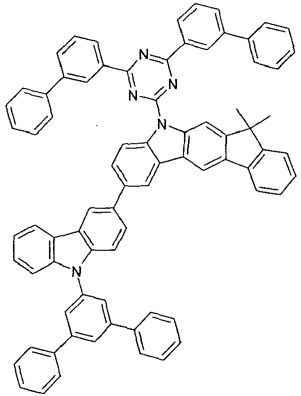 | 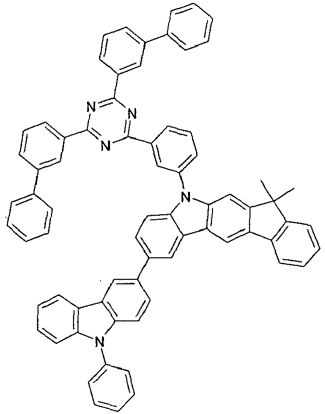 |
| 17 | 18 |
| 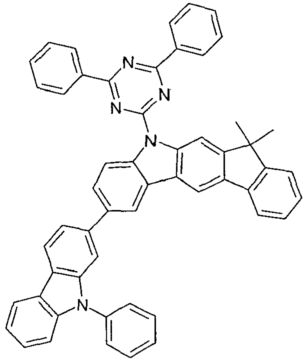 | 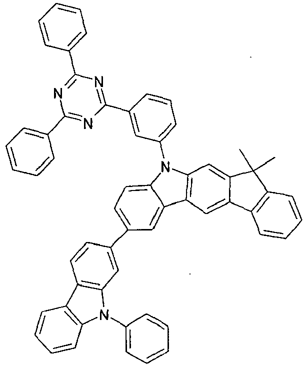 |
| 19 | 20 |
| 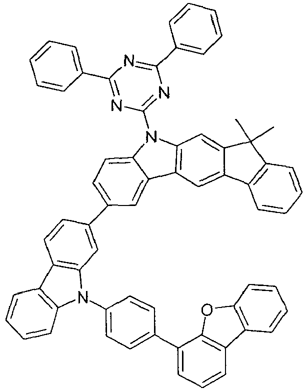 | 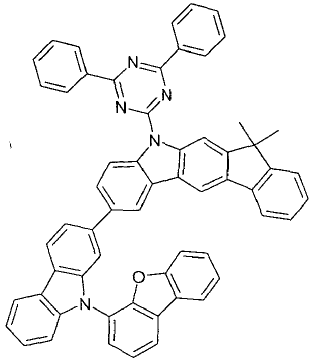 |
| 21 | 22 |
| 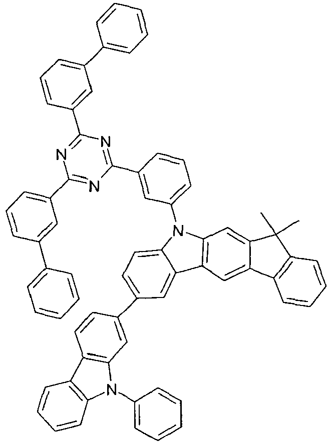 | 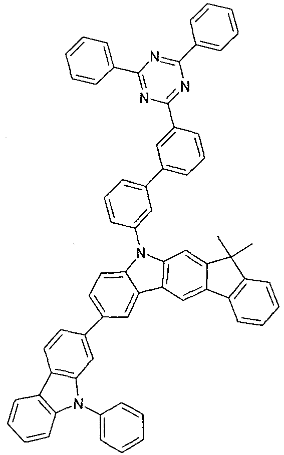 |
| 23 | 24 |
| 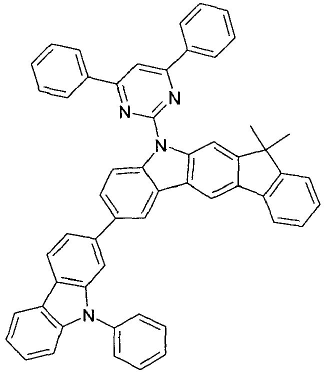 | 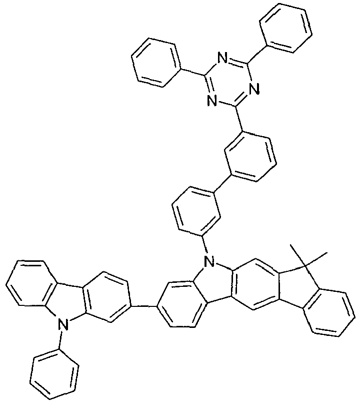 |
| 25 | 26 |
| 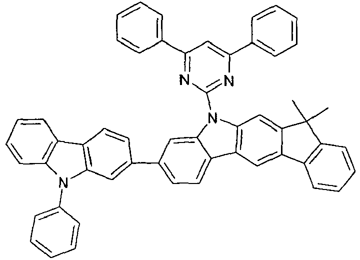 | 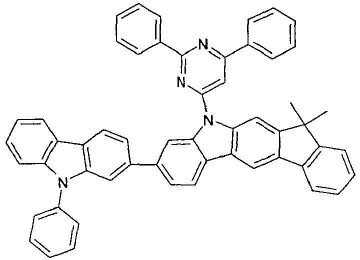 |
| 27 | 28 |
| 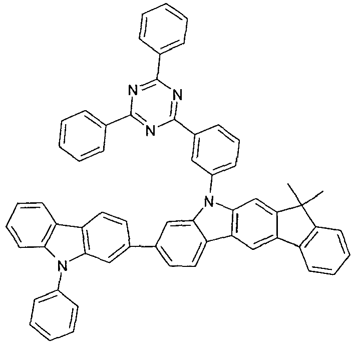 | 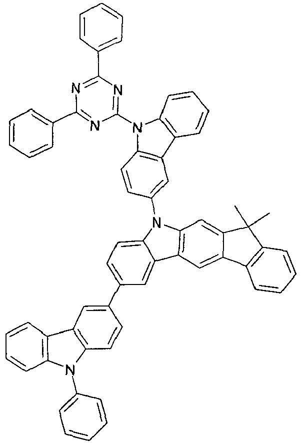 |
| 29 | 30 |
| 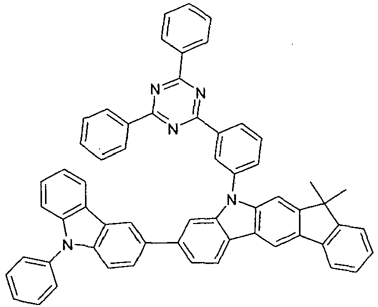 | 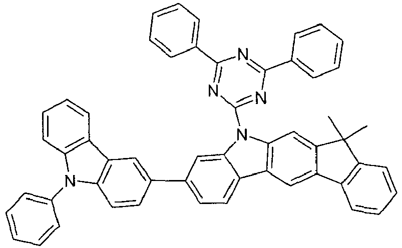 |
| 31 | 32 |
| 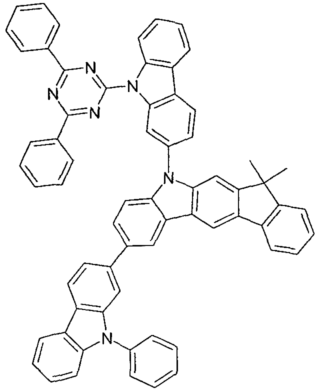 | 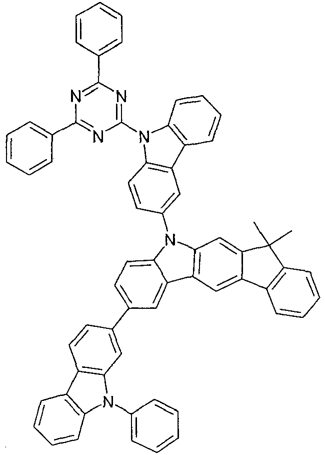 |
| 33 | 34 |
| 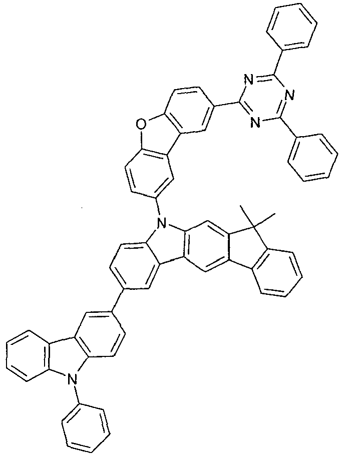 | 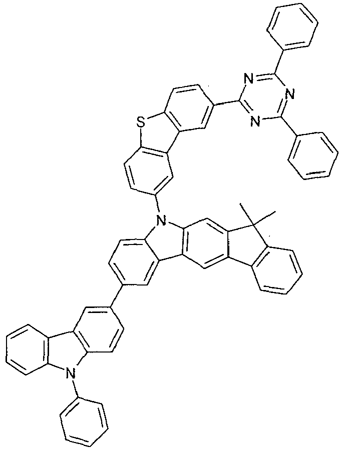 |
| 35 | 36 |
| 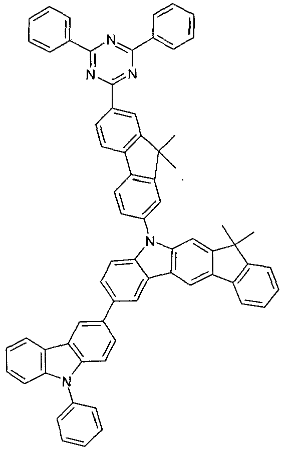 | 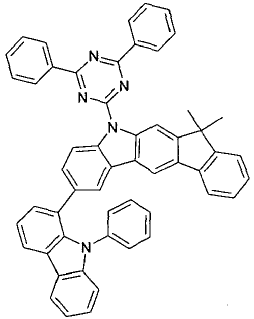 |
| 37 | 38 |
| 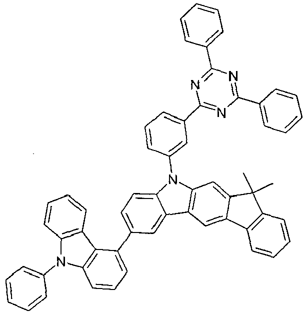 | 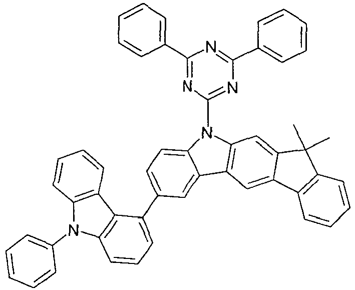 |
| 39 | 40 |
| 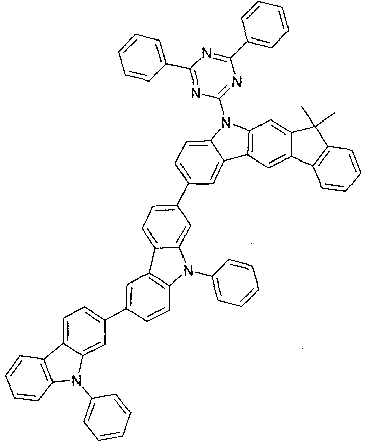 | 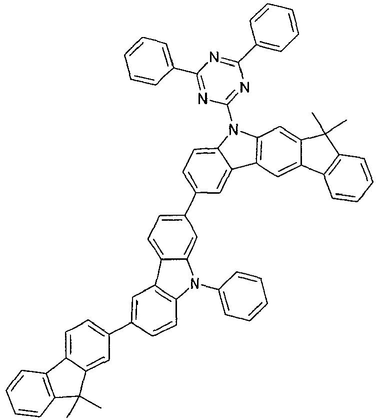 |
| 41 | 42 |
| 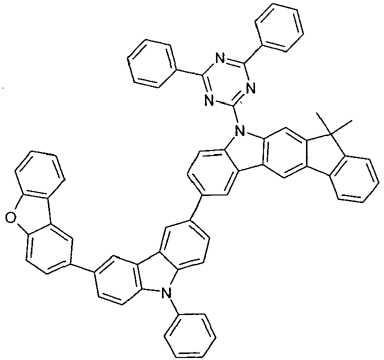 | 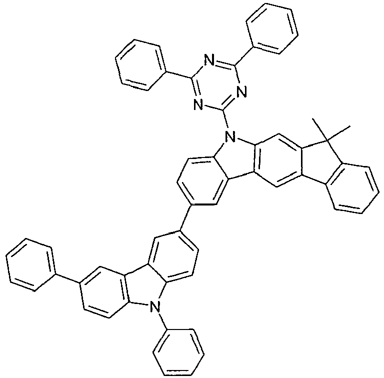 |
| 43 | 44 |
| 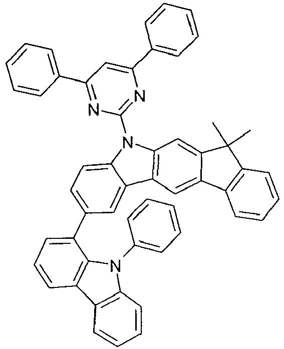 | 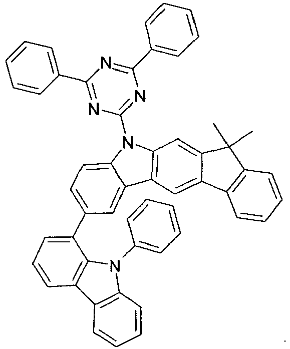 |
| (45) | (46) |
| 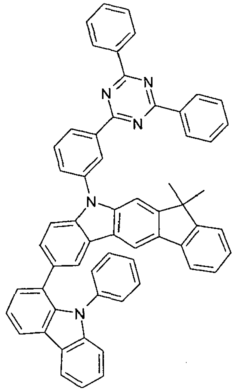 | 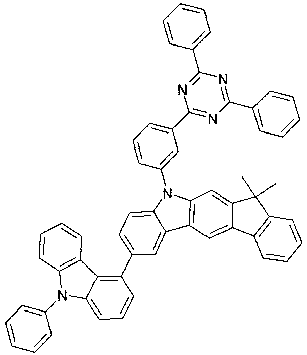 |
| (47) | (48) |
| 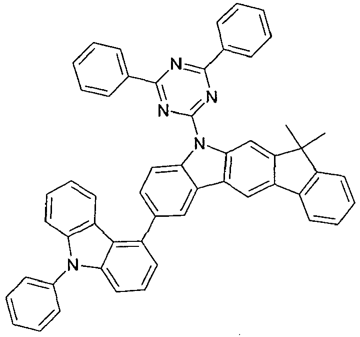 | 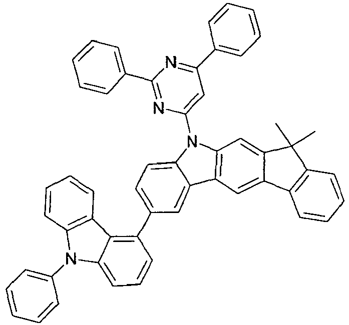 |
| (49) | (50) |
| 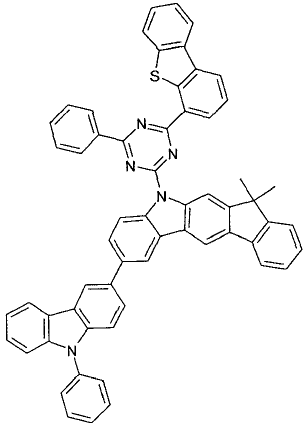 | 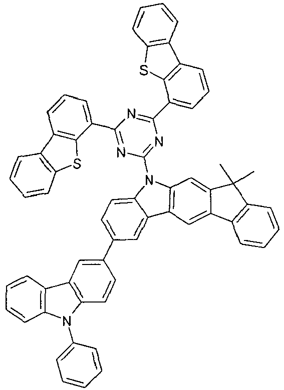 |
| (51) | (52) |
| 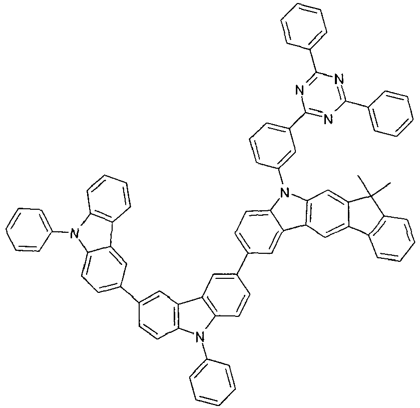 | 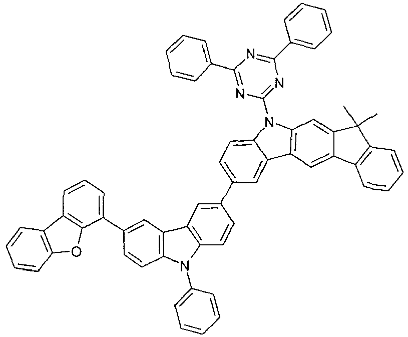 |
| (53) | (54) |
| 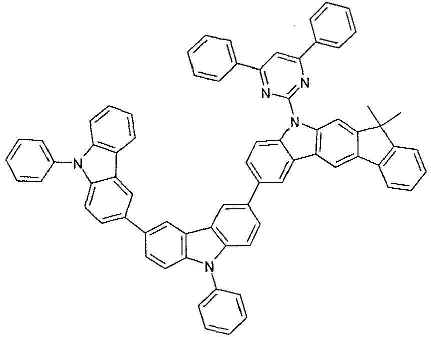 | 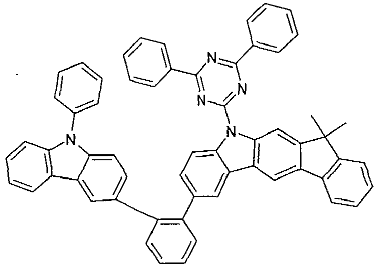 |
| (55) | (56) |
| 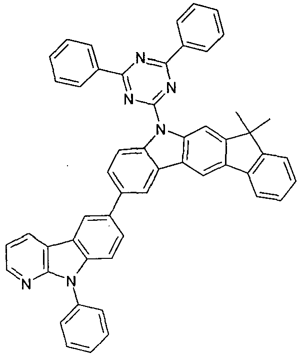 | 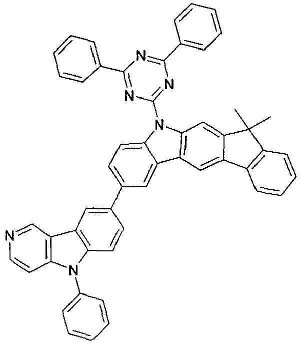 |
| (57) | (58) |
| 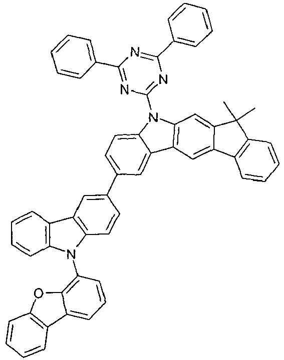 | 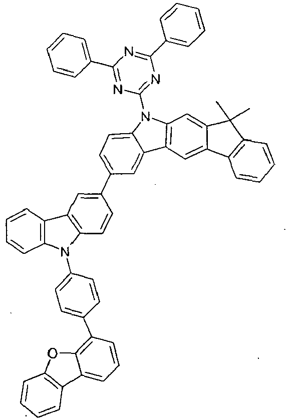 |
| (59) | (60) |
| 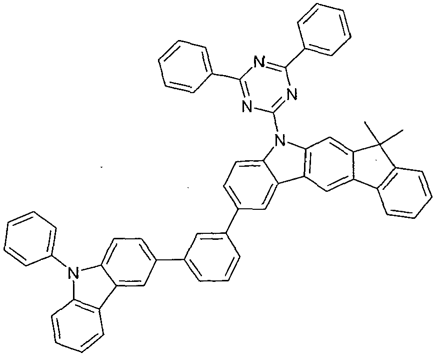 | 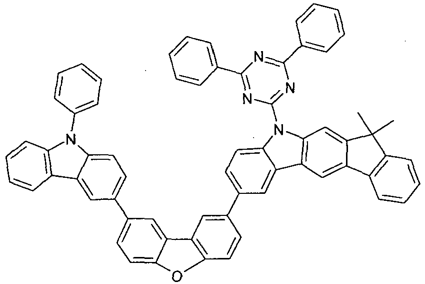 |
| (61) | (62) |
| 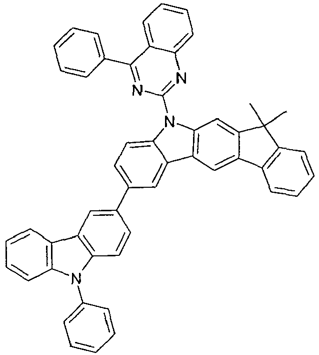 | 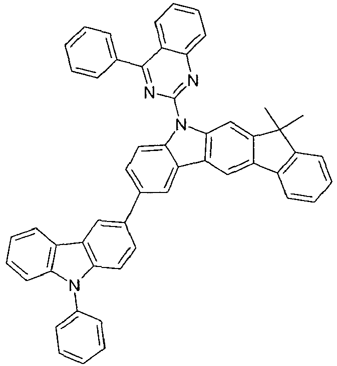 |
| (63) | (64) |
| 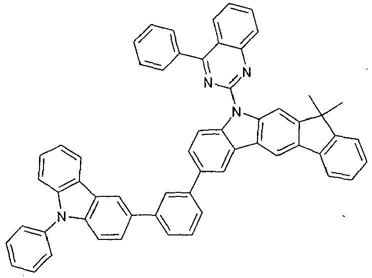 | |
| (65) | |

Die erfindungsgemäßen Verbindungen können mittels bekannter Syntheseschritte der organischen Chemie hergestellt werden. Dazu zählen beispielsweise übergangsmetall-katalysierte Kupplungsreaktionen wie Suzuki- und Buchwald-Kupplung, Bromierungen, und Halogenierungen.

Im Folgenden werden beispielhafte Verfahren zur Herstellung der erfindungsgemäßen Verbindungen vorgestellt. Die gezeigten Verfahren sind besonders geeignet, um die erfindungsgemäßen Verbindungen herzustellen. Es sind jedoch alternative Verfahren denkbar und möglicherweise in bestimmten Fällen vorzuziehen. Entsprechend kann der Fachmann die im Folgenden gezeigten Verfahren im Rahmen seines allgemeinen Fachwissens abwandeln.

Zur Synthese der erfindungsgemäßen Verbindungen wird bevorzugt wie in Schema 1 gezeigt vorgegangen. Die Verbindungen in Schema 1 können an freien Positionen mit beliebigen organischen Resten R substituiert sein.

Dabei wird eine Indenocarbazolverbindung in einem ersten Schritt mit einer halogenierten Arylverbindung enthaltend eine elektronenarme Heteroarylgruppe umgesetzt. Wie solche Indenocarbazolverbindungen hergestellt werden können, ist im Stand der Technik bekannt und durch die Ausführungsbeispiele dieser Anmeldung illustriert. Bevorzugt wird der erste Schritt unter den Bedingungen einer Buchwald-Kupplung durchgeführt. Anschließend wird eine Halogenierung am Indenocarbazol-Grundgerüst durchgeführt. Bevorzugt ist dies eine Bromierung, besonders bevorzugt mit dem Reagenz NBS.

Anschließend an diesen Schritt kann eine Suzuki-Kupplung mit einem Carbazol-Derivat durchgeführt werden, das einen Boronsäure-Substituenten trägt. Alternativ kann das halogenierte Produkt aus dem zweiten Schritt selbst in eine Boronsäure überführt werden und dann in einer Suzuki-Kupplung mit einem halogenierten Carbazol-Derivat umgesetzt werden.

Das erhaltene Produkt kann bereits die Zielverbindung darstellen und entspricht Formel (I). Es können sich jedoch weitere Schritte anschließen, beispielsweise um weitere funktionelle Gruppen oder Reste einzuführen.

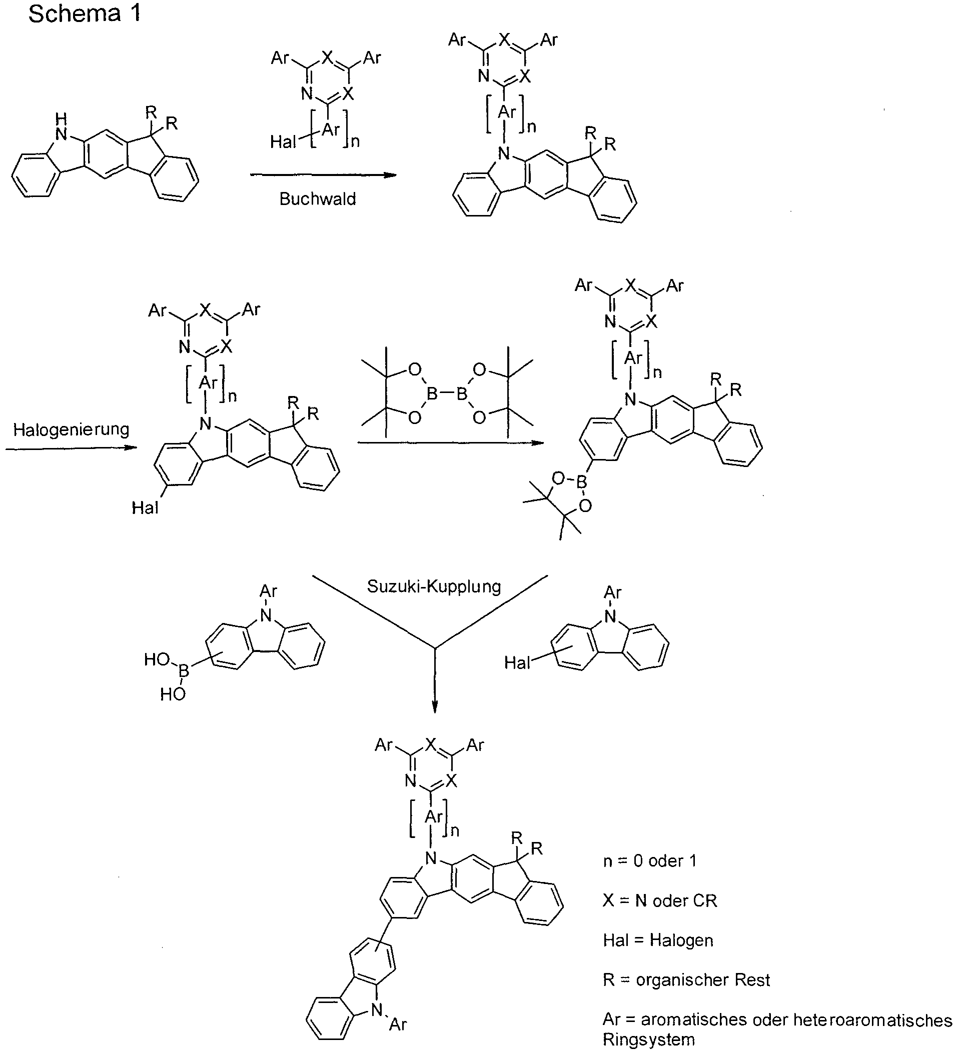

Gegenstand der Erfindung ist somit weiterhin ein Verfahren zur Herstellung einer Verbindung der Formel (I), dadurch gekennzeichnet, dass eine Indenocarbazol-Verbindung mit einem aromatischen oder heteroaromatischen Ringsystem umgesetzt wird, welches eine elektronenarme Heteroarylgruppe enthält, wobei das aromatische oder heteroaromatische Ringsystem an das Stickstoffatom des Indenocarbazols gekuppelt wird. Bevorzugt ist die Reaktion eine Buchwald-Kupplung zwischen einem Indenocarbazol und einem halogenierten aromatischen oder heteroaromatischen Ringsystem.

Weiterhin bevorzugt wird das Reaktionsprodukt anschließend mit einer reaktiven funktionellen Gruppe versehen, beispielsweise durch Halogenierung oder durch Umsetzung zu einer Boronsäure. Weiterhin bevorzugt wird anschließend eine Suzuki-Kupplung mit einem Carbazol-Derivat durchgeführt.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹ oder R² substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 1992/18552), Carbazolen (z. B. gemäß WO 04/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Düsopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die Verbindung gemäß Formel (I) eignet sich für den Einsatz in einer elektronischen Vorrichtung, insbesondere einer organischen Elektrolumineszenzvorrichtung (OLED). Abhängig von der Substitution kann die Verbindung der Formel (I) in unterschiedlichen Funktionen und Schichten eingesetzt werden. Bevorzugt ist die Verwendung als Matrixmaterial in einer emittierenden Schicht, besonders bevorzugt in Kombination mit einem phosphoreszierenden Emitter, und/oder die Verwendung als elektronentransportierendes Material, und/oder die Verwendung als lochblockierendes Material.

Weiterer Gegenstand der Erfindung ist daher die Verwendung einer Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Gegenstand der Erfindung ist weiterhin eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist eine organische Elektrolumineszenzvorrichtung, enthaltend Anode, Kathode und mindestens eine emittierende Schicht, dadurch gekennzeichnet, dass mindestens eine organische Schicht in der Vorrichtung enthalten ist, welche mindestens eine Verbindung gemäß Formel (I) enthält. Bevorzugt ist eine organische Elektrolumineszenzvorrichtung, enthaltend Anode, Kathode und mindestens eine emittierende Schicht, dadurch gekennzeichnet, dass mindestens eine Schicht in der Vorrichtung, gewählt aus emittierenden Schichten, Elektronentransportschichten, Elektroneninjektionsschichten und Lochblockierschichten, mindestens eine Verbindung gemäß Formel (I) enthält.

Unter einer Elektronentransportschicht wird eine beliebige zwischen Kathode und einer emittierenden Schicht angeordnete organische Schicht verstanden, welche elektronentransportierende Eigenschaften hat.

Unter einer Elektroneninjektionsschicht wird eine beliebige zwischen Kathode und einer emittierenden Schicht angeordnete organische Schicht verstanden, welche elektroneninjizierende Eigenschaften hat und unmittelbar an die Kathode angrenzt.

Unter einer Lochblockierschicht wird eine beliebige organische Schicht verstanden, welche sich zwischen emittierender Schicht und Kathode befindet und lochblockierende Eigenschaften aufweist. Bevorzugt befindet sich eine Lochblockierschicht gemäß der vorliegenden Anmeldung zwischen einer emittierenden Schicht und einer elektronentransportierenden Schicht, besonders bevorzugt direkt kathodenseitig angrenzend an eine emittierende Schicht. Materialien der Lochblockierschicht zeichnen sich typischerweise durch ein tiefliegendes HOMO aus.

Außer Kathode, Anode und emittierender Schicht kann die elektronische Vorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die Abfolge der Schichten der elektronischen Vorrichtung ist bevorzugt wie folgt:
-Anode-
-Lochinjektionsschicht-
-Lochtransportschicht-
-optional weitere Lochtransportschichten-
-emittierende Schicht-
-Elektronentransportschicht-
-Elektroneninjektionsschicht-
-Kathode-.
Dabei soll erneut darauf hingewiesen werden, dass nicht alle der genannten Schichten vorhanden sein müssen, und/oder dass zusätzlich weitere Schichten vorhanden sein können.

Die erfindungsgemäße elektronische Vorrichtung kann mehrere emittierende Schichten enthalten. Besonders bevorzugt weisen diese emittierenden Schichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) enthält und wobei die drei Schichten blaue, grüne und orange oder rote Emission zeigen (für den prinzipiellen Aufbau siehe z. B. WO 2005/011013). Alternativ und/oder zusätzlich können die erfindungsgemäßen Verbindungen auch in der Elektronentransportschicht oder in einer anderen Schicht vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Bevorzugt ist die Verbindung gemäß Formel (I) in der elektronischen Vorrichtung als Matrixmaterial in einer emittierenden Schicht enthalten, besonders bevorzugt in Kombination mit einer oder mehreren phosphoreszierenden Emitterverbindungen. Die Emitterverbindungen liegen bevorzugt als Dotanden vor.

Die Begriffe Dotand, Matrixmaterial und phosphoreszierende Emitterverbindung sind dabei wie oben beschrieben definiert.

Bevorzugt in Kombination mit der Verbindung der Formel (I) als Matrixmaterial verwendete Emitterverbindungen sind die weiter unten aufgeführten phosphoreszierenden Emitterverbindungen.

Die emittierende Schicht der elektronischen Vorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung wird die Verbindung gemäß Formel (I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Dotanden umfassen, bevorzugt einen oder mehrere phosphoreszierende Dotanden. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Bevorzugte phosphoreszierende Dotanden zur Verwendung in Mixed-Matrix-Systemen sind die in der obenstehenden Tabelle aufgeführten phosphoreszierenden Dotanden.

Der Anteil des Matrixmaterials in der emittierenden Schicht beträgt in der erfindungsgemäßen elektronischen Vorrichtung bevorzugt zwischen 50.0 und 99.9 Vol.-%, besonders bevorzugt zwischen 80.0 und 99.5 Vol.-% und ganz besonders bevorzugt für fluoreszierende emittierende Schichten zwischen 92.0 und 99.5 Vol.-% sowie für phosphoreszierende emittierende Schichten zwischen 85.0 und 97.0 Vol.-%. Entsprechend beträgt der Anteil des Dotanden bevorzugt zwischen 0.1 und 50.0 Vol.-%, besonders bevorzugt zwischen 0.5 und 20.0 Vol.-% und ganz besonders bevorzugt für fluoreszierende emittierende Schichten zwischen 0.5 und 8.0 Vol.-% sowie für phosphoreszierende emittierende Schichten zwischen 3.0 und 15.0 Vol.-%.

In einer weiteren bevorzugten Ausführungsform der Erfindung wird die Verbindung gemäß Formel (I) als Elektronentransportmaterial in einer Elektronentransportschicht oder Elektroneninjektionsschicht oder Lochblockierschicht eingesetzt.

Im Folgenden sind Materialien angegeben, die bevorzugt in den oben genannten Funktionsschichten der erfindungsgemäßen elektronischen Vorrichtung vorhanden sind.

Als phosphoreszierende Emitter eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Emitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele für phosphoreszierende Emitter können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Die in der folgenden Tabelle aufgeführten Verbindungen stellen besonders geeignete phosphoreszierende Dotanden dar.

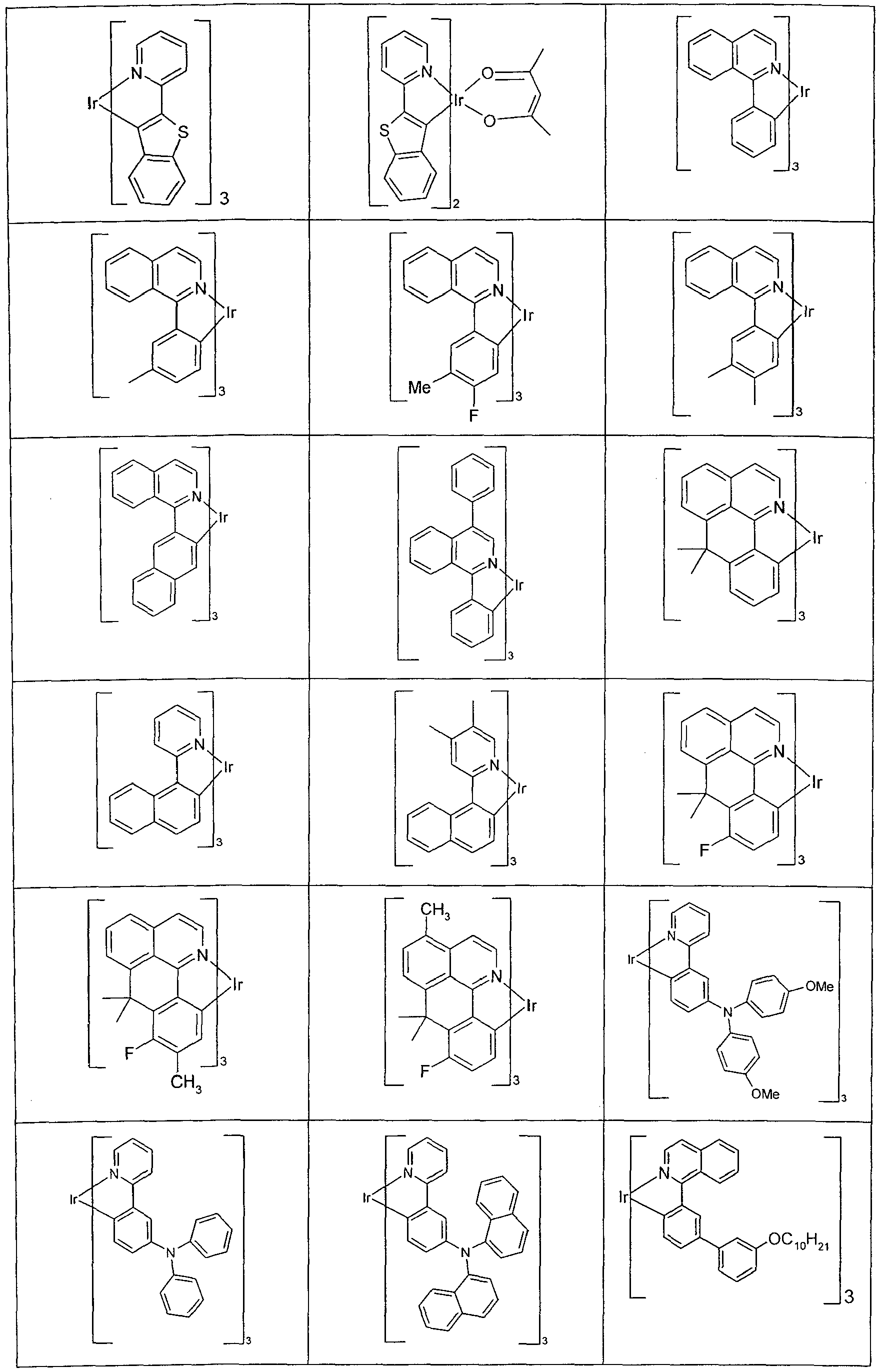

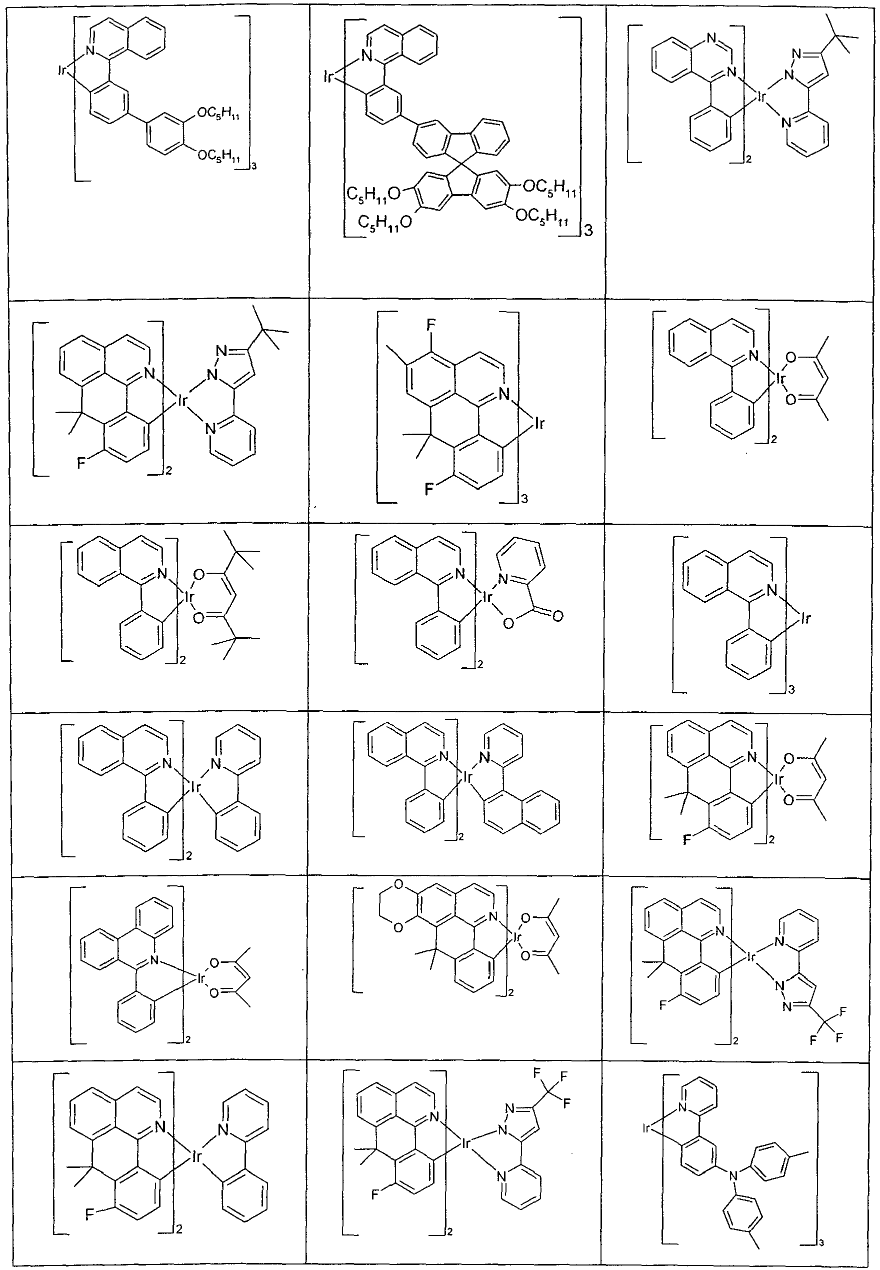

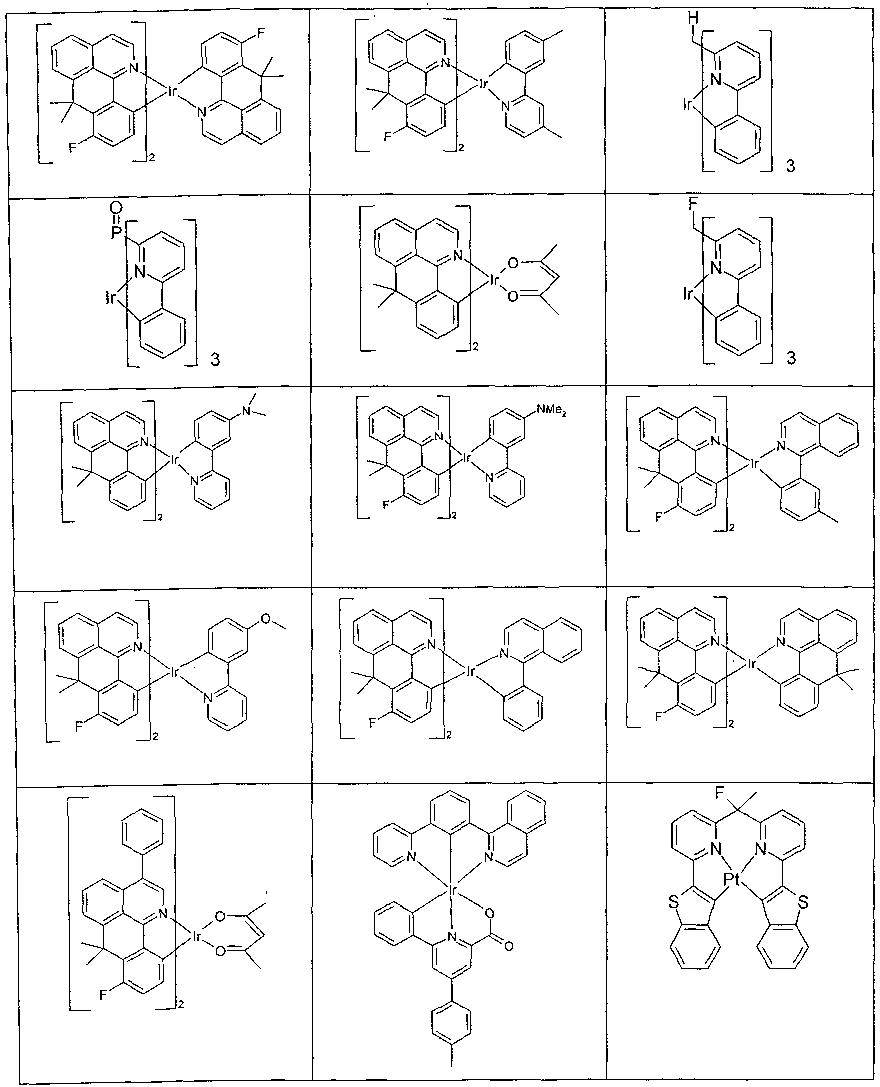

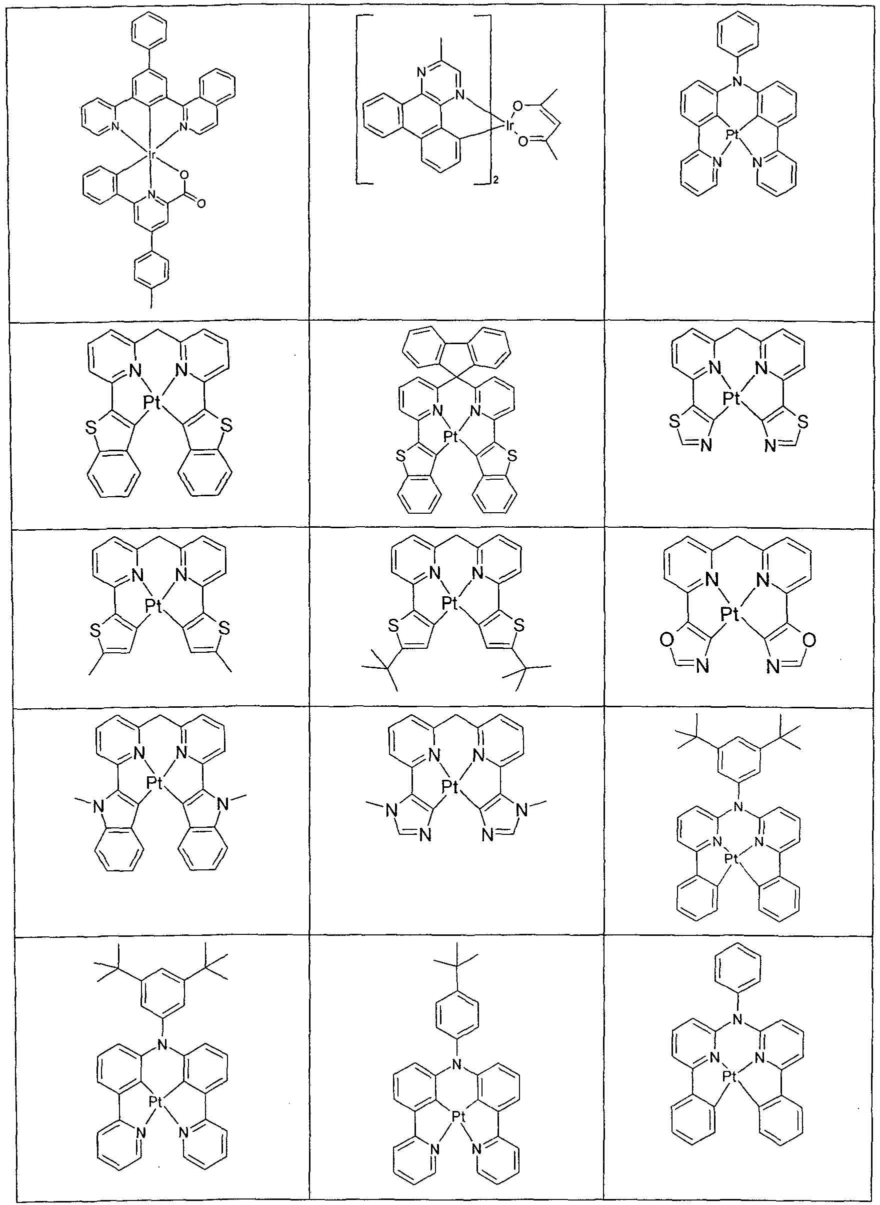

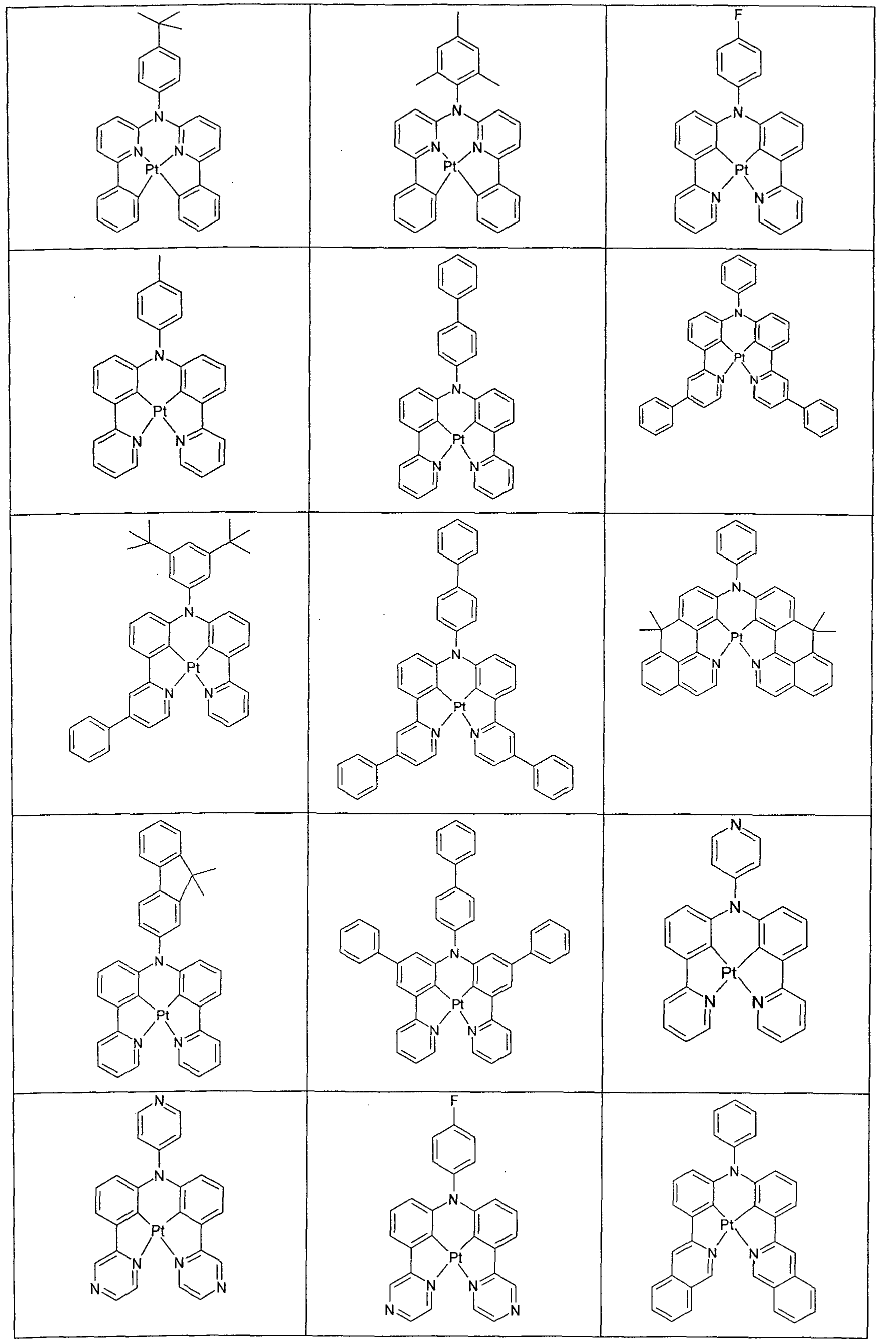

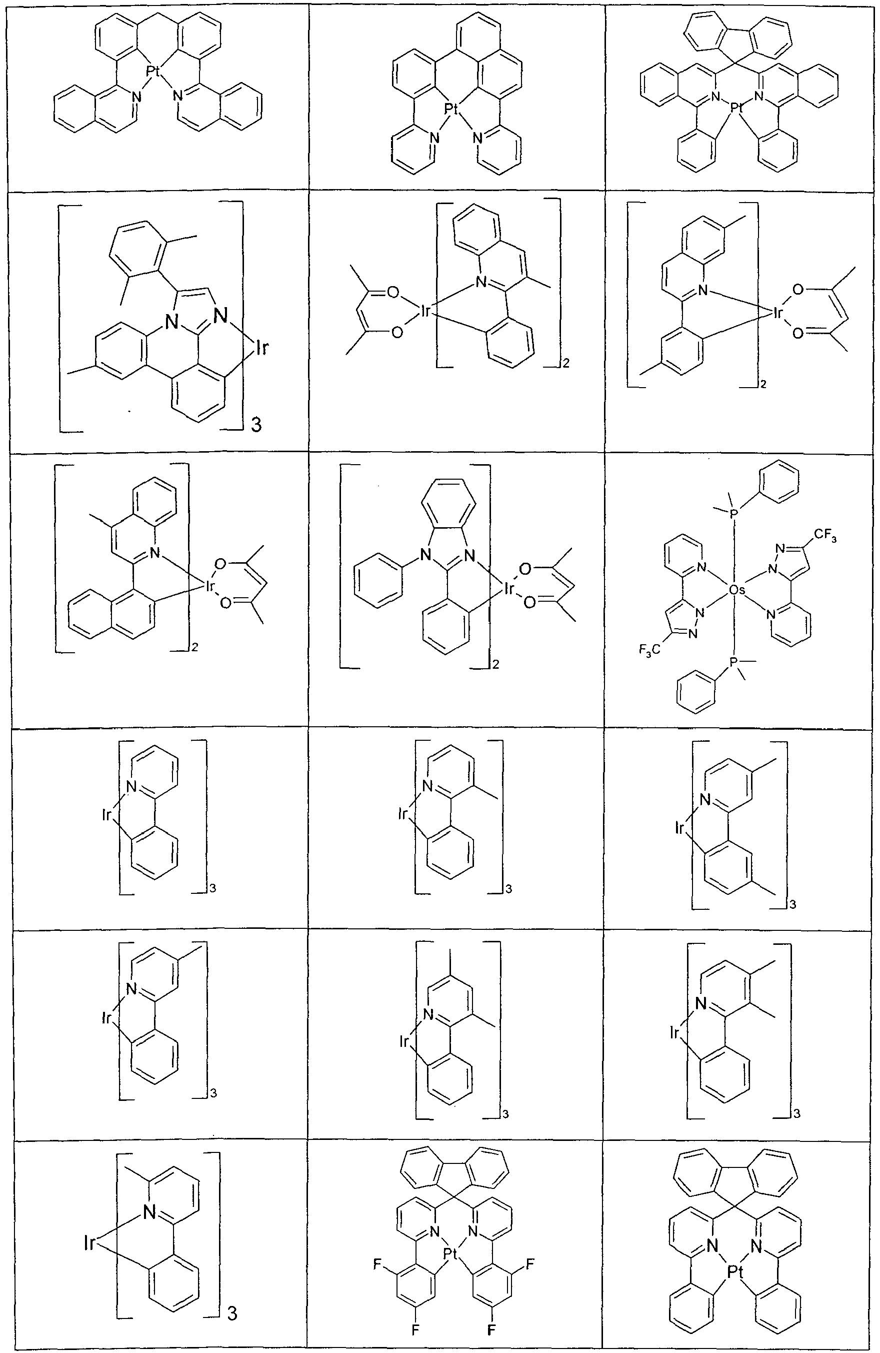

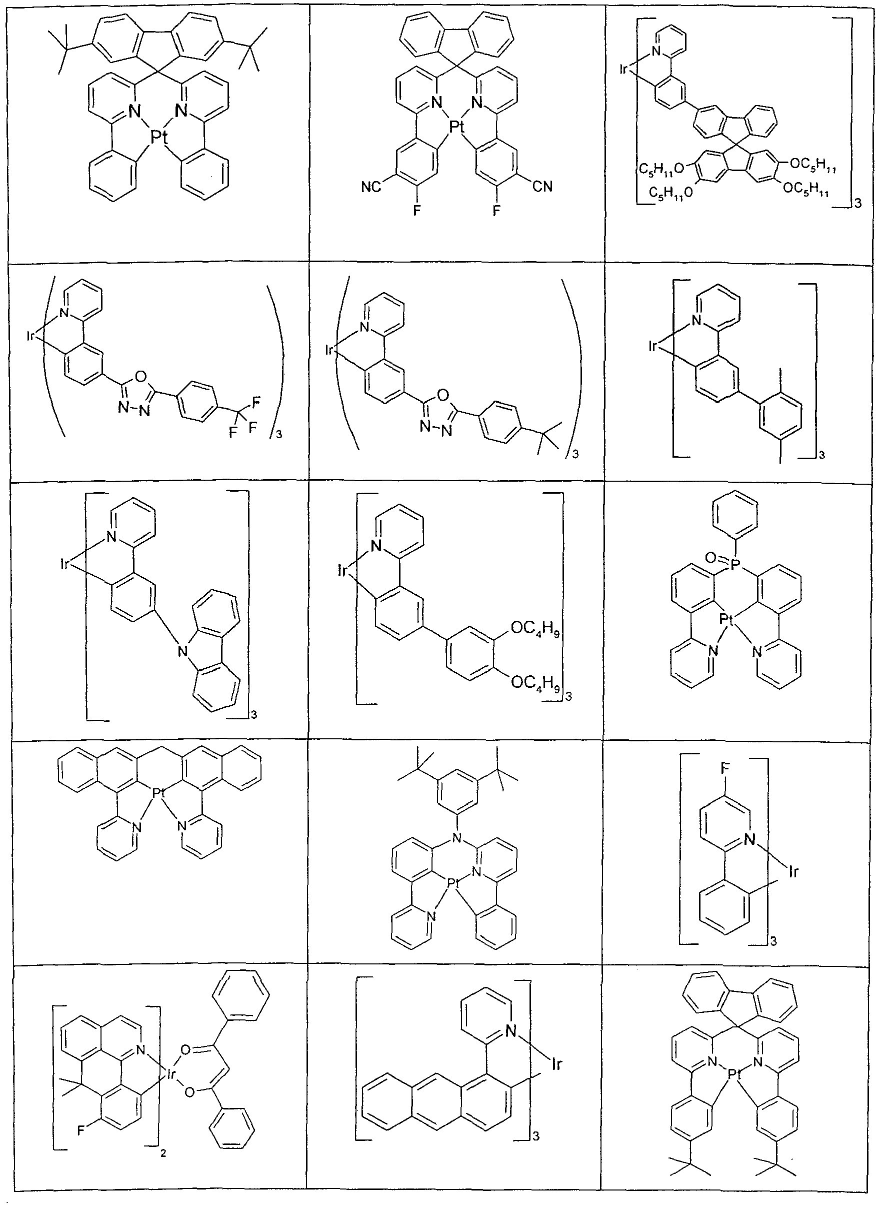

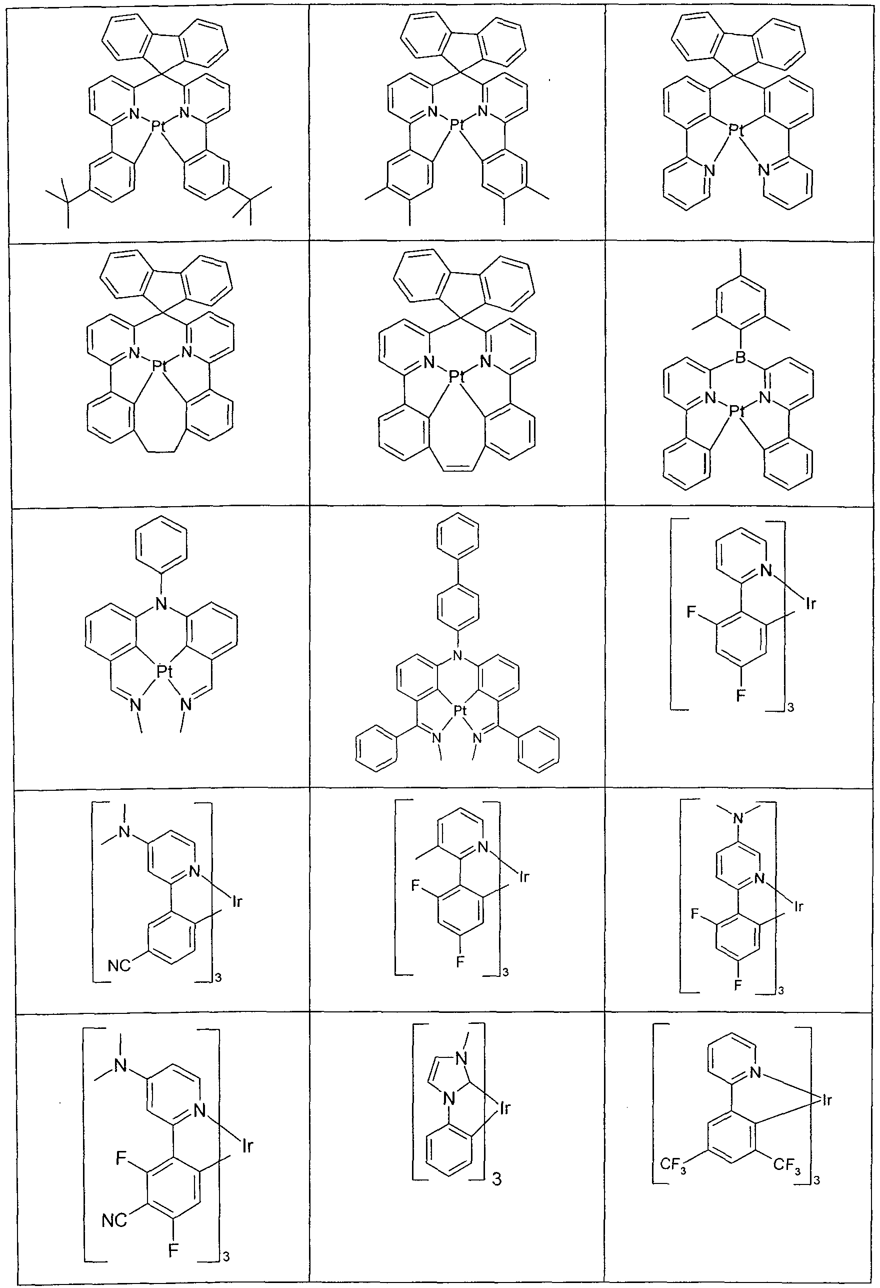

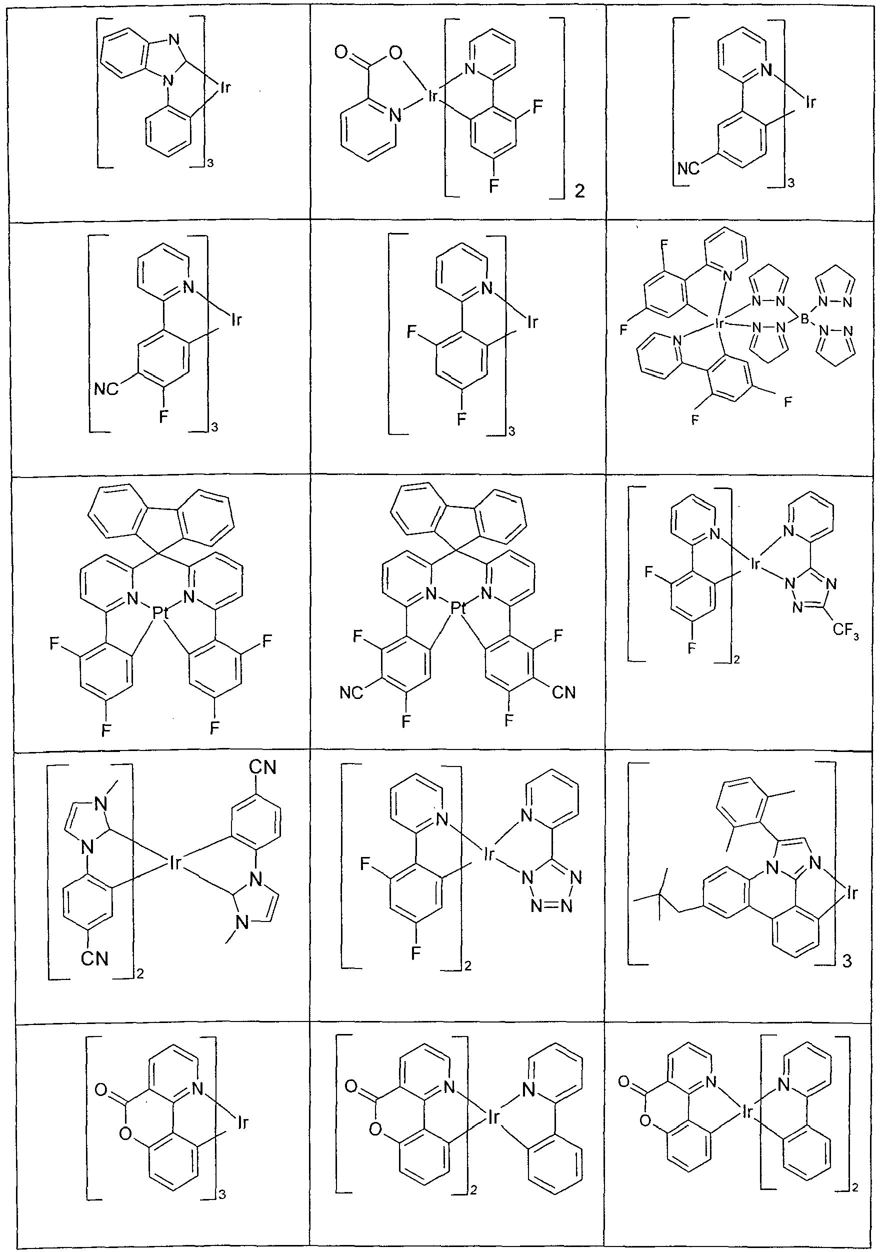

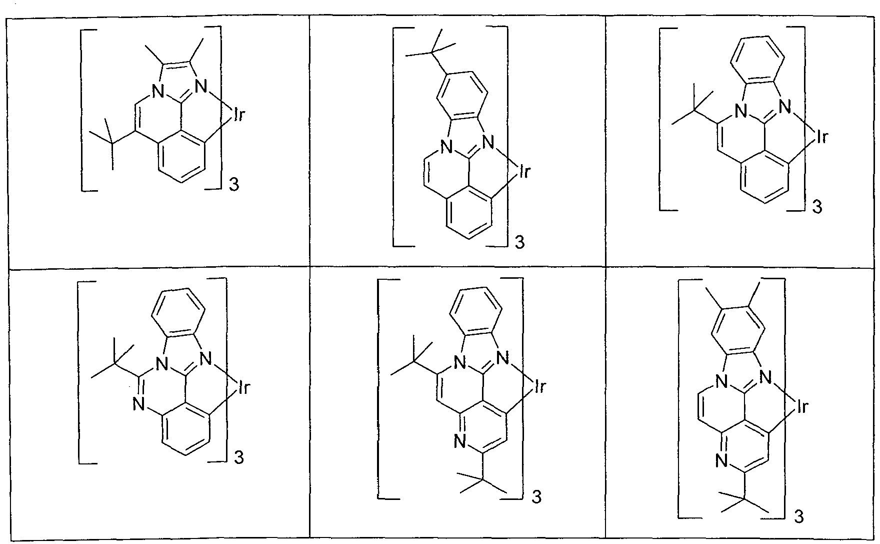

Bevorzugte fluoreszierende Emitter sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Emitterverbindungen sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und der noch nicht offengelegten EP 12004426.8 offenbarten Pyren-Arylamine. Ebenfalls bevorzugt sind die in der noch nicht offengelegten EP 12006239.3 offenbarten Benzoindenofluoren-Amine.

Als Matrixmaterialien, bevorzugt für fluoreszierende Emitter, kommen neben den erfindungsgemäßen Verbindungen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, sowie die in EP 1749809, EP 1905754 und US 2012/0187826 offenbarten Pyren-Verbindungen.

Bevorzugte Matrixmaterialien für phosphoreszierende Emitter sind neben den erfindungsgemäßen Verbindungen aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAIq, Diazasilol- und Tetraazasilol-Derivate, z. B. gemäß WO 2010/054729 und DiazaphospholDerivate, z. B. gemäß WO 2010/054730.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können neben den erfindungsgemäßen Verbindungen alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Bevorzugt sind als Lochtransportmaterialien, die in einer Lochtransport-, Lochinjektions- oder Elektronenblockierschicht in der erfindungsgemäßen Elektrolumineszenzvorrichtung verwendet werden können, Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß WO 2013/083216) und Dihydroacridin-Derivate (z. B. gemäß WO 2012/150001).

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

Die folgenden Ausführungsbeispiele dienen zur Erläuterung der Erfindung. Sie sollen nicht einschränkend ausgelegt werden.

### A) Synthese der erfindungsgemäßen Verbindungen

Die nachfolgenden Synthesen werden, sofern nicht anders angegeben, unter einer Schutzgasatmosphäre in getrockneten Lösungsmitteln durchgeführt. Die erfindungsgemäßen Verbindungen können mittels dem Fachmann bekannten Syntheseverfahren dargestellt werden.

### I) Synthesen von Vorstufen

### Beispiel 1: 2-Chlor-4,6-diphenyl-pyrimidin

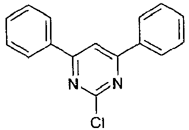

75 g (0,41 mmol) 1,3,5-Trichlorpyrimidin, 100 g (0,82 mol) Phenylboronsäure und 625 ml 4M NaHCO₃-Lösung werden in 2,5 L Ethylenglycoldimethylether suspendiert. Zu dieser Suspension werden 2,3 g (10,23 mmol) Pd(OAc)₂ und 10,35 g (34 mmol) P(o-Tol)₃ gegeben und die Reaktionsmischung wird 16 h unter Rückfluss erhitzt. Das Gemisch wird im Anschluss zwischen Essigester und Wasser verteilt, die organische Phase dreimal mit Wasser gewaschen und über Na₂SO₄ getrocknet und einrotiert. Der verbleibende Rückstand wird aus Heptan/Toluol umkristallisiert. Die Ausbeute beträgt 43 g (0,15 mol, 38%).

### Beispiel 2: 4-(2-Brom-phenyl)-2,6-diphenyl-pyrimidin

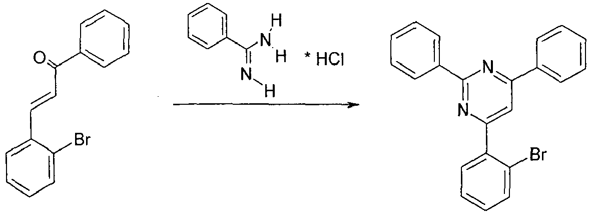

23 g (409 mmol) Kaliumhydroxid werden in 500 mL Ethanol gelöst, bei Raumtemperatur mit 40 g (255 mmol) Benzamid-hydrochlorid und 129 g (452 mmol) (3-(Bromphenyl)-1-phenyl-2-propen-1-on, gelöst in 500 ml Ethanol, versetzt und 3 h unter Rückfluss gerührt. Nach Kühlung auf Raumtemperatur wird der ausgefallene Feststoff abgesaugt, mit etwas Ethanol gewaschen und getrocknet. Es verbleiben 55 g (129 mmol), 50 % des Produkts, in Form farbloser Kristalle.

### Beispiel 3: 2,4-Bis-biphenyl-3-yl-6-chloro-[1,3,5]triazin

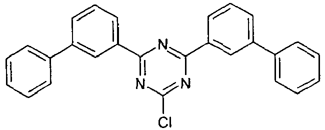

5,2 g Magnesium (0,215 mol) werden in einem 500 mL Vierhalskolben vorgelegt, und eine Lösung aus 50 g Brombiphenyl (214 mmol) in 200 ml THF wird langsam zugetropft. Die Reaktionsmischung wird 1,5 h zum Sieden erhitzt und anschließend auf Raumtemperatur abgekühlt. In einem zweiten Kolben wird Cyanchlorid (17,2 g, 93 mmol) in 150 ml THF vorgelegt und auf 0°C gekühlt. Bei dieser Temperatur wird dann das abgekühlte Grignard-Reagenz zugetropft und 12 h bei RT gerührt. Nach dieser Zeit wird die Reaktionsmischung mit 150 mL HCl versetzt und die wässrige Phase dreimal mit Dichlormethan extrahiert. Die vereinigten organischen Phasen werden mit Wasser gewaschen, über Na₂SO₄ getrocknet und eingeengt. Der Rückstand wird aus EtOH umkristallisiert. Die Ausbeute beträgt 32,8 g (78 mmol, 84%).

### Beispiel 4: 2-Chlor-4,6-bis-[1,1';3',1"]terphenyl-5'-yl-[1,3,5]triazin

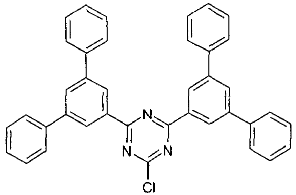

3,93 g Magnesium (162 mmol) werden in einem 500 mL Vierhalskolben vorgelegt, und eine Lösung aus 50 g 5'-Brom-[1,1';3',1"]terphenyl (162 mmol) in 150 ml THF wird langsam zugetropft. Die Reaktionsmischung wird 1,5 h zum Sieden erhitzt und anschließend auf Raumtemperatur abgekühlt. In einem zweiten Kolben wird Cyanchlorid (13 g, 70 mmol) in 150 ml THF vorgelegt und auf 0 °C gekühlt. Bei dieser Temperatur wird das abgekühlte Grignard-Reagenz zugetropft und 12 h bei RT gerührt. Nach dieser Zeit wird die Reaktionsmischung mit 150 mL HCl versetzt und die wässrige Phase dreimal mit Dichlormethan extrahiert. Die vereinigten organischen Phasen werden mit Wasser gewaschen, über Na₂SO₄ getrocknet und eingeengt. Der Rückstand wird aus EtOH umkristallisiert. Die Ausbeute beträgt 27,8 g (49 mol, 70%).

### Beispiel 5: 2-Chlor-4,6-bis-(3-([3,1';5,1"]terphen-1-yl)-phen-1-yl)-1,3,5-triazin

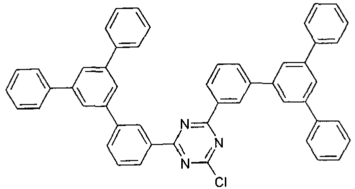

2,0 g Magnesium (81 mmol) werden in einem 500 mL Vierhalskolben vorgelegt, und eine Lösung von 31,2 g 5'-(3-Bromphenyl)-[1,1';3',1"]terphenyl (81 mmol) in 100 ml THF wird langsam zugetropft. Die Reaktionsmischung wird 1,5 h zum Sieden erhitzt und anschließend auf Raumtemperatur abgekühlt. In einem zweiten Kolben wird Cyanchlorid (6,4 g, 35 mmol) in 50 ml THF vorgelegt und auf 0 °C gekühlt. Bei dieser Temperatur wird das abgekühlte Grignard-Reagenz zugetropft und 12 h bei Raumtemperatur gerührt. Nach dieser Zeit wird die Reaktionsmischung mit 150 mL HCl versetzt, die wässrige Phase dreimal mit Dichlormethan extrahiert. Die vereinigten organischen Phasen werden mit Wasser gewaschen und über Na₂SO₄ getrocknet und eingeengt. Der Rückstand wird aus Toluol umkristallisiert. Die Ausbeute beträgt 6,8 g (9,4 mmol, 28%).

### Beispiel 6: 3-Bromo-9-[1,1';3',1"]terphenyl-5'-yl-9H-carbazol

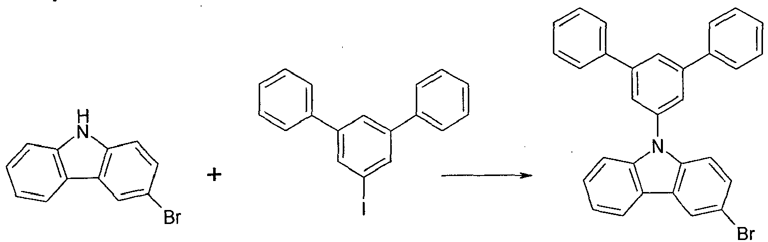

10 g (41 mmol) 3-Bromo-9H-Carbazol (CAS 86-74-8) und 16 g (45 mmol, 1.1 eq) 5'-Iodo-[1,1';3',1"]terphenyl werden zusammen mit 9.2 g (160 mmol, 4 eq) Kaliumhydroxid, 300 mg (1.6 mmol, 0.04 eq) 1,10-Phenanthrolin und 160 mg (1.6 mmol, 0.04 eq) Kupfer(I)-iodid in 250 ml p-Xylol gelöst und unter Rückfluss erhitzt. Nach beendeter Reaktion wird das Gemisch dreimal mit Wasser extrahiert und die organische Phase über Natriumsulfat getrocknet, das Lösungsmittel im Vakuum entfernt und der erhaltene Feststoff mittels Säulenchromatogrpahie (Ethylacetat/Heptan) aufgereinigt. Es werden 17 g (36 mmol, 88%) des gewünschten Produkts erhalten.

Analog können folgende Verbindungen erhalten werden:

| **Beispiel** | **Edukt 1** | **Edukt 2** | **Produkt** |
|---|---|---|---|
| **6a** | 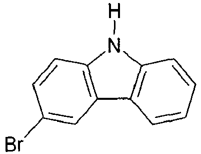 | 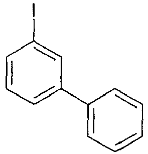 | 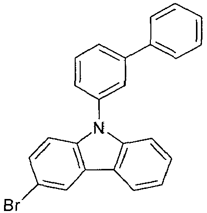 |
| | CAS 86-74-8 | CAS 20442-79-9 | |
| **6b** | 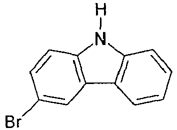 | 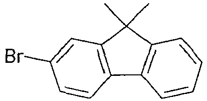 | 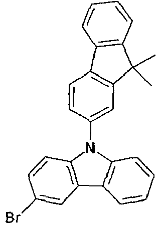 |
| | CAS 86-74-8 | CAS 28320-31-2 | |
| **6c** | 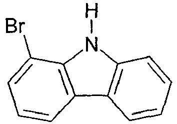 | 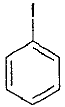 | 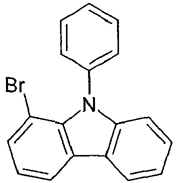 |
| | CAS 16807-11-7 | CAS 591-50-4 | |

| **Beispiel** | **Ausbeute** |
|---|---|
| **6a** | 94% |
| **6b** | 91% |
| **6c** | 96% |

### Beispiel 7: 9-(9,9-Dimethyl-9H-fluoren-2-yl)-9H-carbazol-3-Boronsäure

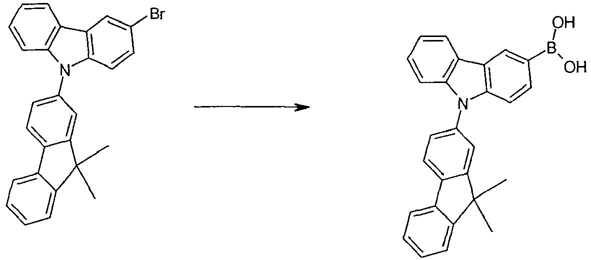

22,3 g (51 mmol) 3-Bromo-9-(9,9-dimethyl-9H-fluoren-2-yl)-9H-carbazol werden in 600 mL trockenem THF gelöst und auf -78°C gekühlt. Bei dieser Temperatur wird mit 26.2 mL (65.7 mmol / 2.5 M in Hexan) n-BuLi innerhalb von ca. 5 min. versetzt und anschließend für 2.5h bei -78°C nachgerührt. Bei dieser Temperatur wird mit 7.3 mL (65.7 mmol) Borsäuretrimethylester möglichst zügig versetzt und die Reaktion langsam auf RT kommen gelassen (ca. 18h). Die Reaktionslösung wird mit Wasser gewaschen und der ausgefallene Feststoff und die organische Phase werden mit Toluol azeotrop getrocknet. Das Rohprodukt wird aus Toluol/Methylenchlorid bei ca. 40°C ausgerührt und abgesaugt. Man erhält 17,5 g (85%) des Produktes als weißen Feststoff.

Analog können folgende Verbindungen erhalten werden:

| | **Edukt** | **Produkt** | **Ausbeute** |
|---|---|---|---|
| 7a | 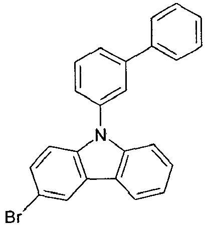 | 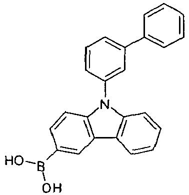 | 79% |
| 7b | 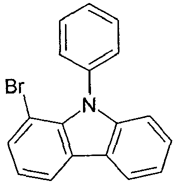 | 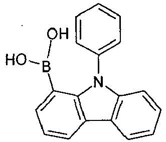 | 81% |

### Beispiel 8: 3-(5-Bromo-biphenyl-3-yl)-9-phenyl-9H-carbazol

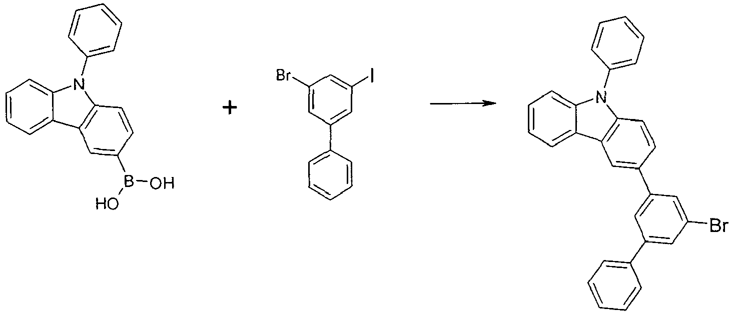

15.5 g (43.3 mmol) 3-Bromo-5-iodo-biphenyl und 13,7 g (48 mmol) (9-Phenyl-9H-carbazol-3-yl)-boronsäure werden in 80 mL Toluol gelöst und entgast. Es wird mit 281 mL einer entgasten 2M K₂CO₃-Lösung und mit 2.5 g (2.2 mmol) Pd(OAc)₂ versetzt. Die Reaktionsmischung wird anschließend bei 80 °C für 48 h unter Schutzgasatmosphäre gerührt. Die abgekühlte Lösung wird mit Toluol verdünnt, mehrmals mit Wasser gewaschen, getrocknet und eingeengt. Das Produkt wird via Säulenchromatographie an Kieselgel mit Toluol/Heptan (1:2) gereinigt. Die Reinheit beträgt 98 %. Ausbeute: 17,6 g (37 mmol, 78 %) der Theorie.

Analog können folgende Verbindungen erhalten werden:

| | **Edukt 1** | **Edukt 2** | **Produkt** |
|---|---|---|---|
| 8a | 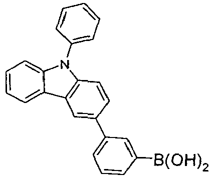 | 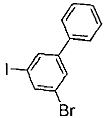 | 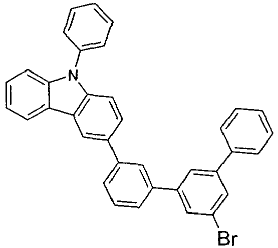 |
| | 854952-60-6 | 136649-44-0 | |
| 8b | 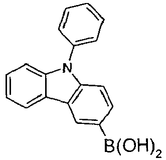 | 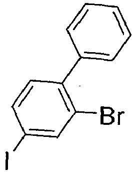 | 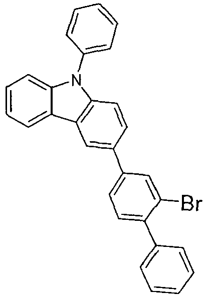 |
| | 854952-58-2 | 900806-53-3 | |
| 8c | 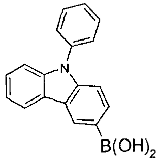 | 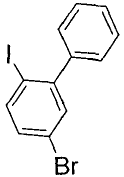 | 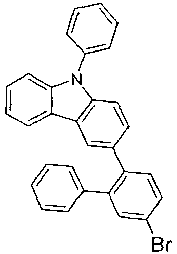 |
| | 854952-58-2 | 4510-78-5 | |
| 8d | 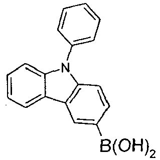 | 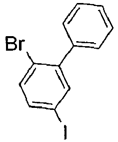 | 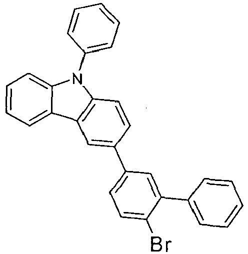 |
| | 854952-58-2 | 1226452-23-8 | |

| | **Ausbeute** |
|---|---|
| 8a | 70% |
| 8b | 69% |
| 8c | 68% |
| 8d | 83% |

### Beispiel 9: 3-(5-Boronsäure-biphenyl-3-yl)-9-phenyl-9H-carbazol

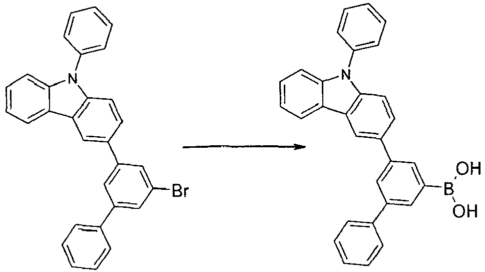

Eine auf -78 °C gekühlte Lösung von 128 g (270 mmol) 3-(5-Bromo-biphenyl-3-yl)-9-phenyl-9-H-carbazol in 1500 ml Diethylether wird tropfenweise mit 110 ml (276 mmol) n-Butyllithium (2.5 M in Hexan) versetzt. Die Reaktionsmischung wird 30 min. bei -78 °C gerührt. Man lässt auf Raumtemperatur kommen, kühlt erneut auf -78 °C und versetzt dann schnell mit einer Mischung von 40 ml (351 mmol) Trimethylborat in 50 ml Diethylether. Nach Erwärmen auf -10 °C wird mit 135 ml 2 N Salzsäure hydrolysiert. Die organische Phase wird abgetrennt, mit Wasser gewaschen, über Natriumsulfat getrocknet und zur Trockene eingeengt. Der Rückstand wird in 300 ml n-Heptan aufgenommen, der farblose Feststoff wird abgesaugt, mit n-Heptan gewaschen und im Vakuum getrocknet. Ausbeute: 112 g (256 mmol), 95 % d. Th.

Analog können folgende Verbindungen erhalten werden:

| | **Edukt** | **Produkt** | **Ausbeute** |
|---|---|---|---|
| 9a | 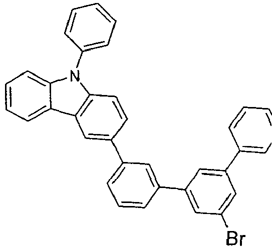 | 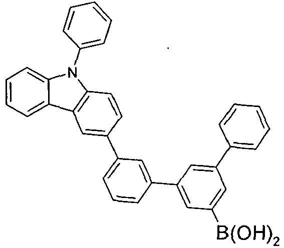 | 64% |
| 9b | 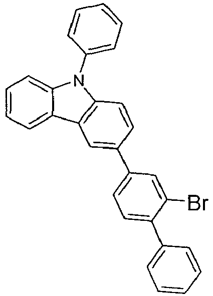 | 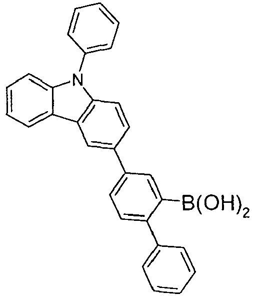 | 60% |
| 9c | 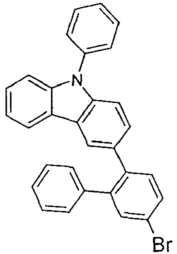 | 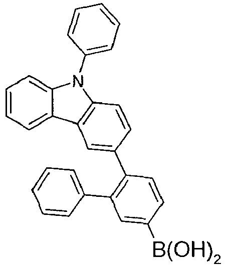 | 63% |
| 9d | 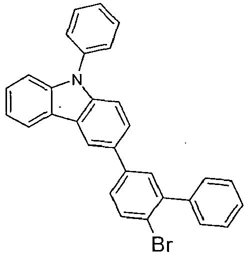 | 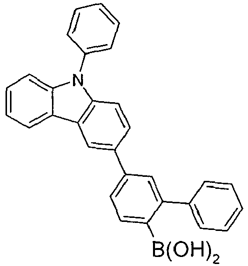 | 69% |
| 9e | 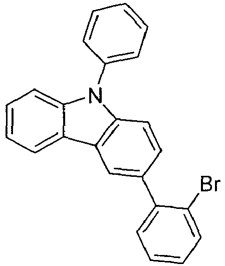 | 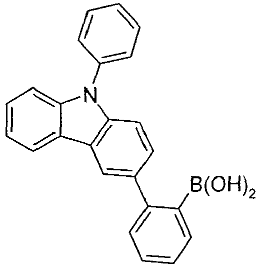 | 59% |
| | 1190100-35-6 | | |
| 9f | 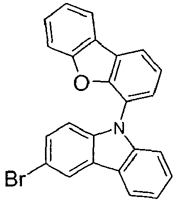 | 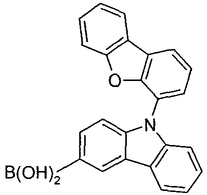 | 83% |
| | 1345970-20-8 | | |

### Beispiel 10e:

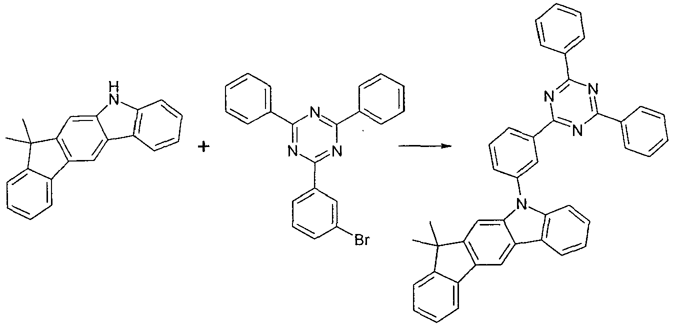

Unter Schutzgas werden 22 g (79,8 mmol) 2,12-Dimethyl-10-phenyl-10,12-dihydro-10-aza-indeno[2,1-b]fluoren, 34 g (87 mmol) des Triazins und 15,9 ml (15,9 mmol) 1 Mol/l Tri-tert-butylphosphin, 1,79 g (7,9 mmol) Palladiumacetat in 120 ml p-Xylol suspendiert. Die Reaktionsmischung wird 16 h unter Rückfluss erhitzt. Nach Erkalten wird die organische Phase abgetrennt, dreimal mit 200 mL Wasser gewaschen und anschließend zur Trockene eingeengt. Der Rückstand wird mit Toluol heiß extrahiert, aus Toluol umkristallisiert und abschließend im Hochvakuum sublimiert, die Reinheit beträgt 99,9%. Ausbeute: 38 g (64 mmol), 80 % der Theorie.

Analog werden folgende Verbindungen erhalten:

| **Bsp.** | **Edukt 1** | **Edukt 2** | **Produkt** |
|---|---|---|---|
| 10c | 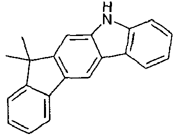 | 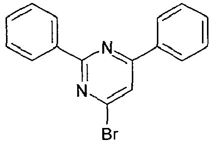 | 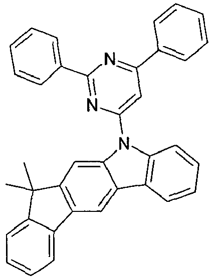 |
| | | 40734-4-5 | |
| 10d | 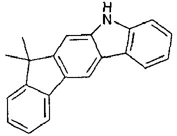 | 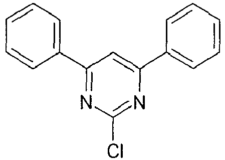 | 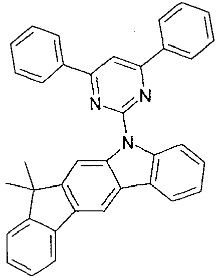 |
| 10f | 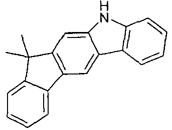 | 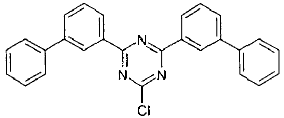 | 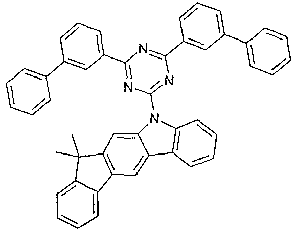 |
| 10g | 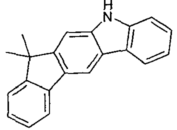 | 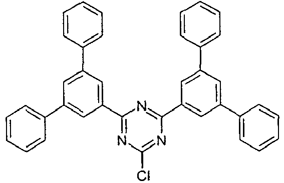 | 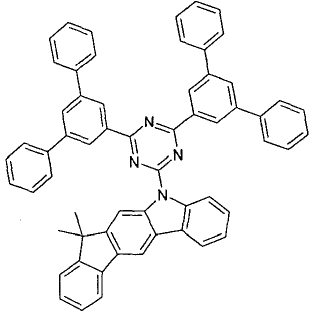 |
| 10h | 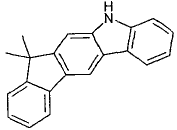 | 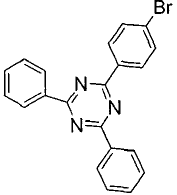 | 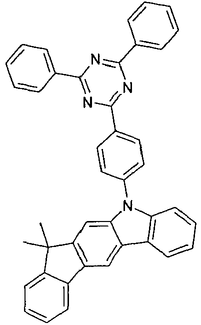 |
| | | 23449-08-3 | |
| 10i | 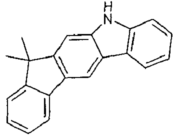 | 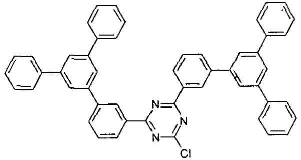 | 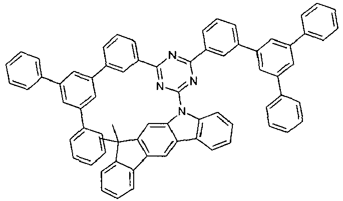 |

| **Bsp.** | **Ausbeute** |
|---|---|
| 10c | 81% |
| 10d | 76% |
| 10f | 72% |
| 10g | 70% |
| 10h | 85% |
| 10i | 80% |

### Beispiel 11e:

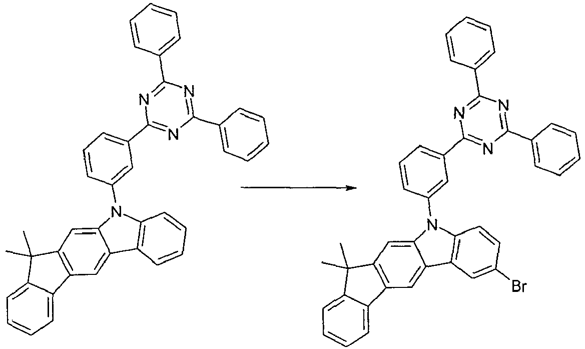

Es werden 23 g (40.18 mmol) des Edukts in 800 mL Acetonitril suspendiert und bei -20°C portionsweise mit 7.15 g (40.18 mmol) N-Bromsuccinimid versetzt, so dass die Reaktionstemperatur nicht über -20°C steigt. Es wird für 18h nachgerührt und die Temperatur wird dabei auf RT kommen gelassen. Die Reaktionsmischung wird anschließend einrotiert, mit Dichlormethan gelöst und mit Wasser gewaschen. Es wird getrocknet, eingeengt und anschließend aus Toluol bis auf eine Reinheit von 99.3% umkristallisiert. Man erhält 20,8 g (80%) des Produktes als weißen Feststoff.

Analog werden folgende Verbindungen erhalten:

| **Bsp.** | **Edukt** | **Produkt** |
|---|---|---|
| 11c | 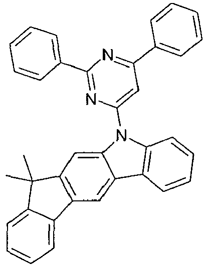 | 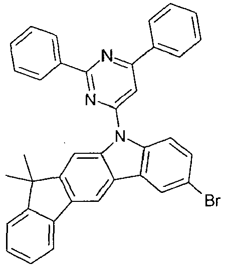 |
| 11d | 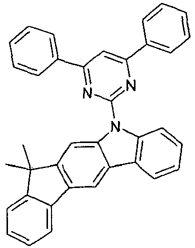 | 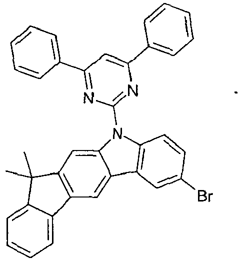 |
| 11f | 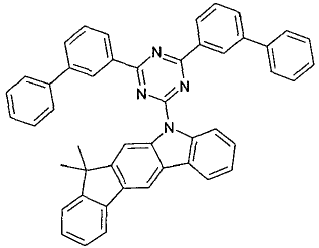 | 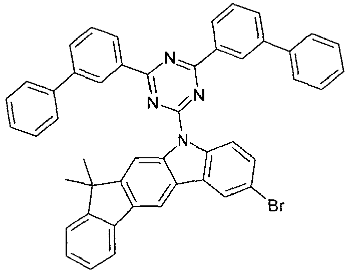 |
| 11g | 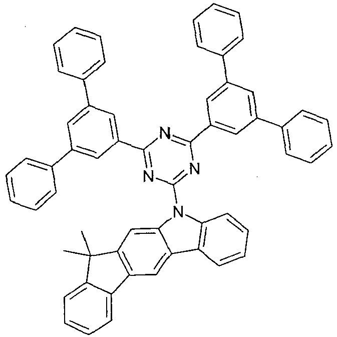 | 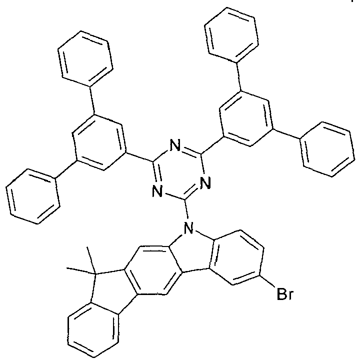 |
| 11h | 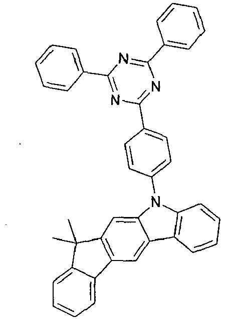 | 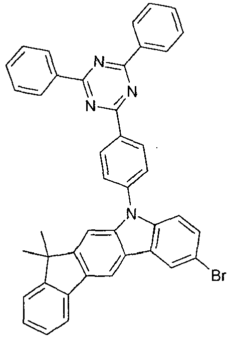 |
| 11i | 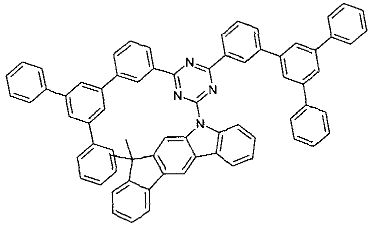 | 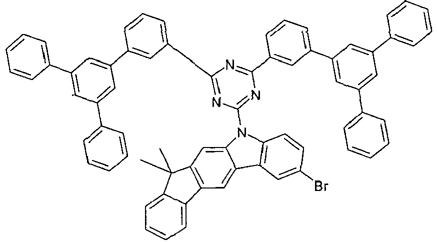 |

| **Bsp.** | **Ausbeute** |
|---|---|
| 11c | 81% |
| 11d | 76% |
| 11f | 72% |
| 11g | 70% |
| 11h | 85% |
| 11i | 80% |

### II) Synthese von erfindungsgemäßen Verbindungen

### Beispiel 12f:

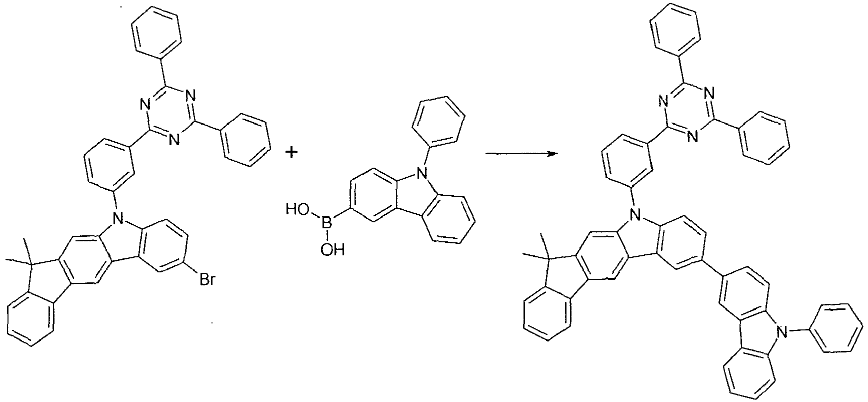

28,9 g (43,3 mmol) des Edukts und 13,7 g (48 mmol) 9-Phenyl-9H-carbazol-3-boronsäure werden in 80 mL Toluol gelöst und entgast. Es wird mit 281 mL einer entgasten Lösung von 2M K₂CO₃ und mit 2,5 g (2,2 mmol) Pd(OAc)₂ versetzt. Die Reaktionsmischung wird anschließend bei 80 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt und abschließend im Hochvakuum (p = 5 x 10⁻⁷ mbar) sublimiert. Die Reinheit beträgt 99,9%. Ausbeute: 28 g (31 mmol), 80 % der Theorie.

| **Bsp.** | **Edukt 1** | **Edukt 2** | **Produkt** |
|---|---|---|---|
| 12c | 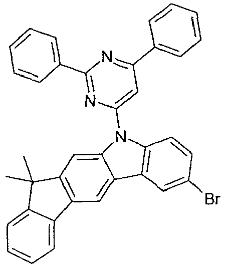 | 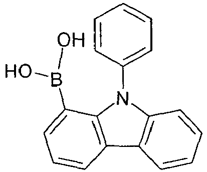 | 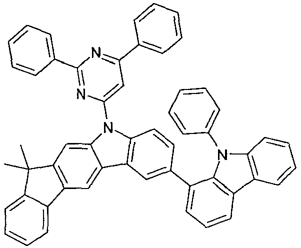 |
| 12e | 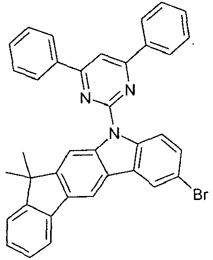 | 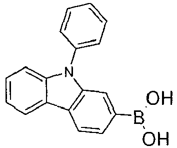 | 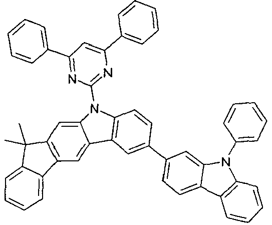 |
| | | 1001911-63-2 | |
| 12g | 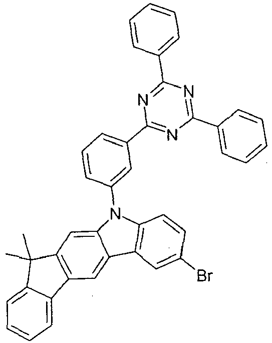 | 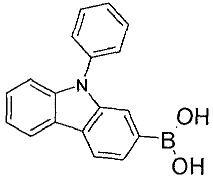 | 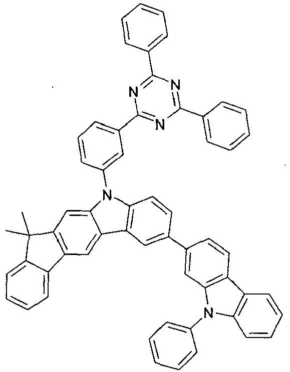 |
| | | 1001911-63-2 | |
| 12h | 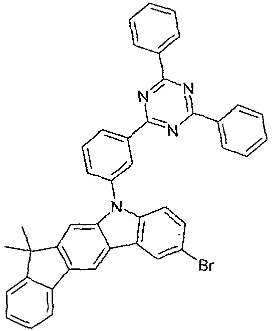 | 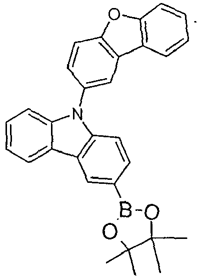 | 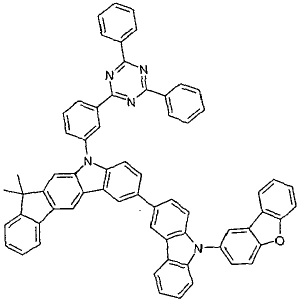 |
| | | 1338488-91-7 | |
| 12j | 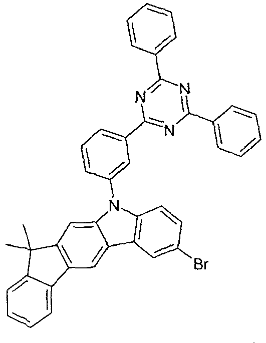 | 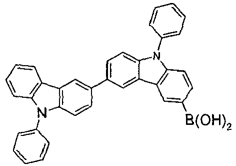 | 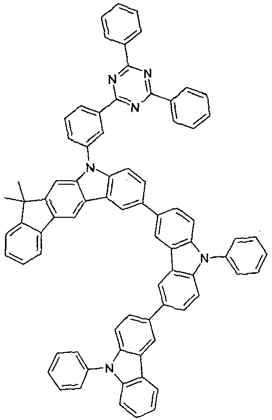 |
| | | 918137-86-7 | |
| 12i | 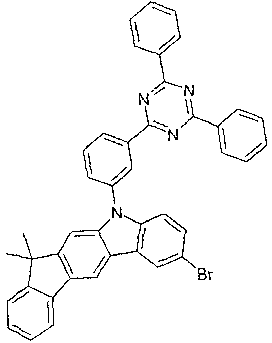 | 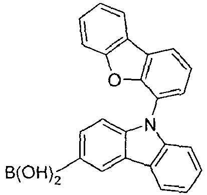 | 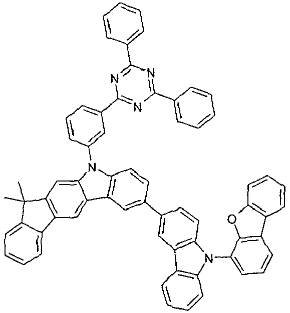 |
| 12k | 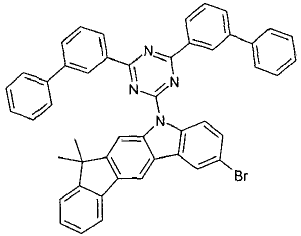 | 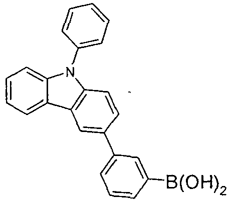 | 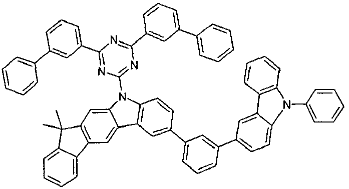 |
| | | 854952-60-6 | |
| 12l | 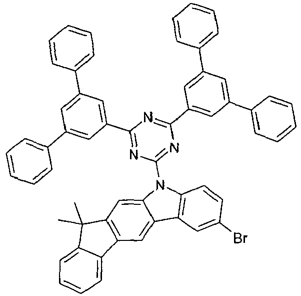 | 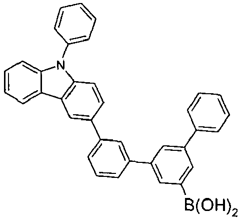 | 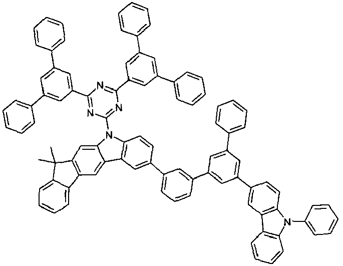 |
| 12m | 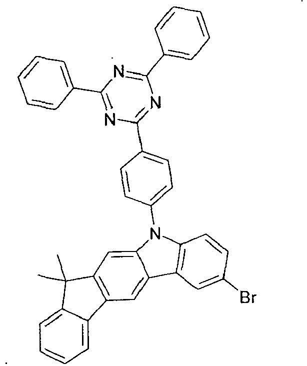 | 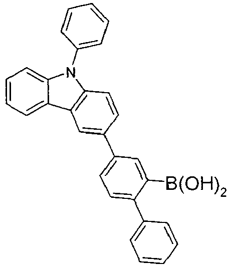 | 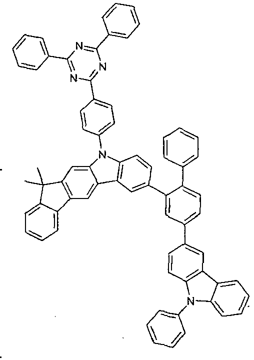 |
| 12o | 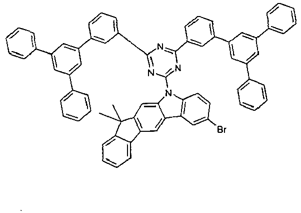 | 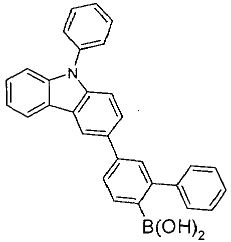 | 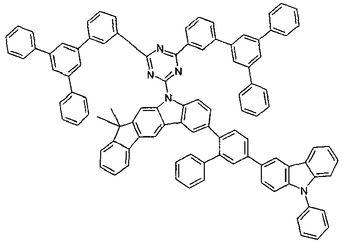 |
| 12u | 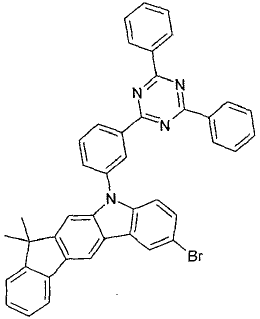 | 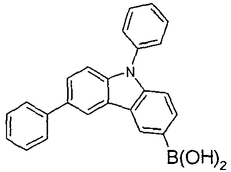 | 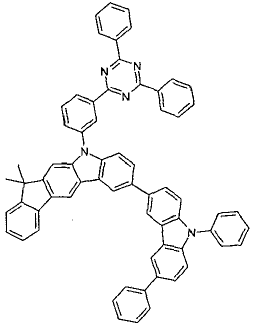 |
| | | 1133058-06-6 | |
| 12v | 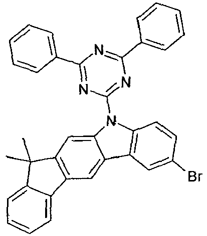 | 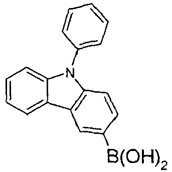 | 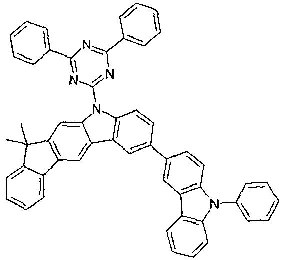 |
| | | 854952-58-2 | |
| | 1346010-98-7 | | |
| 12w | 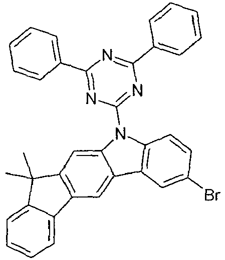 | 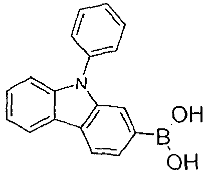 | 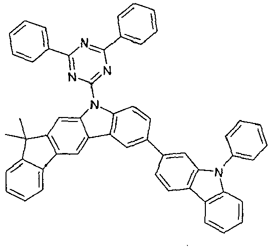 |
| | | 1001911-63-2 | |
| | 1346010-98-7 | | |

| **Bsp.** | **Ausbeute** |
|---|---|
| 12c | 81% |
| 12e | 76% |
| 12g | 79% |
| 12h | 82% |
| 12j | 80% |
| 12i | 76% |
| 12k | 72% |
| 12l | 70% |
| 12m | 85% |
| 12o | 80% |
| 12u | 79% |
| 12v | 83% |
| 12w | 78% |

### B) Verwendung der erfindungsgemäßen Verbindungen in OLEDs

In den folgenden Beispielen V1 bis E9 (siehe Tabellen 1 und 2) werden die Daten verschiedener OLEDs vorgestellt. Beispiele V1 bis V7 sind dabei Vergleichsbeispiele, Beispiele E1 bis E9 sind dabei erfindungsgemäße Beispiele.

Die OLEDs werden wie folgt hergestellt:

Gereinigte Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylenedioxythiophene) poly(styrenesulfonate), bezogen als CLEVIOS™ P VP Al 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / Zwischenschicht (IL) / Elektronenblockierschicht (EBL) / Emissionsschicht (EML) / Optionale Lochblockierschicht (HBL) / Elektronentransportschicht (ETL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie SdT1:TEG1 (90%:10%) bedeutet hierbei, dass das Material SdT1 in einem Volumenanteil von 90% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Stromdichte von 1000 cd/m² bestimmt und daraus die CIE 1931 x und y Farbkoordinaten berechnet. Die Angabe U10 in Tabelle 2 bezeichnet die Spannung, die für eine Stromdichte von 10 mA/cm² benötigt wird. SE10 und LE10 bezeichnen die Strom- bzw. Leistungseffizienz, die bei 10 mA/cm² erreicht werden. EQE10 schließlich bezeichnet die externe Quanteneffizienz bei einer Betriebsstromdichte von 10 mA/cm².

Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 cd/m² und L1 = 80% in Tabelle 2 bedeutet, dass die in Spalte LD angegebene Lebensdauer der Zeit entspricht, nach der die Anfangsleuchtdichte von 4000 cd/m² auf 3200 cd/m² absinkt. Analog bedeutet L0;j0 = 20mA/cm², L1 = 70%, dass die Leuchtdichte bei Betrieb mit 20mA/cm² nach der Zeit LD auf 70% ihres Anfangswertes absinkt.

Die Daten, die mit den verschiedenen hergestellten OLEDs gemessen werden, sind in Tabelle 2 zusammengefasst.

Im Folgenden werden einige der Beispiele näher erläutert, um die Vorteile der erfindungsgemäßen Verbindungen zu verdeutlichen. Alle verwendeten Materialien sind entweder als OLED-Materialien im Stand der Technik bekannt, oder ihre Synthese und Verwendung ist in der vorliegenden Anmeldung beschrieben.

### Verwendung von erfindungsgemäßen Verbindungen als Matrixmaterialien in phosphoreszierenden OLEDs

In allen gemessenen Vorrichtungen werden bei Verwendung der erfindungsgemäßen Verbindungen als Matrixmaterialien in der emittierenden Schicht in Kombination mit Triplett-Emittern (Beispiele E1 bis E9, Verbindungen 12f,12g, 12h, 12k, 12m, 12w) sehr gute Leistungsdaten, insbesondere Lebensdauer und Effizienz, erhalten.

Verglichen mit der Verbindung SdT5 gemäß dem Stand der Technik werden mit der erfindungsgemäßen Verbindung 12w deutlich verbesserte Effizienz und Lebensdauer erzielt (Beispiele V7, E7). Die Verbindung 12w unterscheidet sich von der Verbindung SdT5 durch das Merkmal, dass die CR₂-Gruppe und die NR-Gruppe des Indenocarbazols cis zueinander stehen und nicht trans.

Bei Einsatz der Verbindung SdT4, die eine Pyrimidin-Gruppe am Carbazol enthält, erhält man gute Leistungsdaten. Bei Austausch der Pyrimidin-Gruppe durch eine Triazin-Gruppe, wie in Verbindung 12f, ergibt sich allerdings eine um 75% verbesserte Lebensdauer (Beispiele V3, E1).

Das Vorhandensein einer Triazin-Gruppe gegenüber einer Pyrimidingruppe, gebunden über einen Linker an die IndenocarbazolEinheit, ergibt daher überraschenderweise eine deutliche Verbesserung der Leistungsdaten.

Bei Verwendung der Verbindung 12w wird gegenüber der Vergleichsverbindung SdT3, die eine Phenylgruppe anstelle einer Triazingruppe trägt, eine Verbesserung der Leistungsdaten erhalten (E7, V6). Insbesondere verbessert sich die Lebensdauer deutlich und die Effizienz leicht.

Die Verwendung einer Triazingruppe anstelle einer Phenylgruppe als Substituent am Stickstoff der Indenocarbazolgruppe verbessert also deutlich die Leistungsdaten der OLED enthaltend die Verbindung.

Weiterhin werden mit den erfindungsgemäßen Verbindungen bereits bei geringen Emitterkonzentrationen, wie beispielsweise 5 Vol-%, sehr gute Leistungsdaten erhalten, insbesondere sehr gute Lebensdauer und sehr gute Effizienz (Beispiele E2, E8 und E9).

Geringe Emitterkonzentrationen in der emittierenden Schicht sind für OLEDs von hohem Interesse, da die in den Emitter verwendeten Metalle wie Iridium und Platin sehr selten und damit teuer sind. Weiterhin sind die Komplexe häufig wenig temperaturstabil, so dass langsam aufgedampft werden muss. Die Aufdampfzeiten können verringert werden, wenn Konzentrationen und damit die Mengen an aufzudampfendem Emitter geringer sind.

Die Leistungsdaten sind mit den erfindungsgemäßen Vorrichtungen gegenüber den Vergleichsvorrichtungen deutlich verbessert, wie die folgenden Vergleiche zeigen. Mit Verbindung 12w erhält man beispielsweise bei leicht verbesserter Effizienz eine deutlich verbesserte Lebensdauer gegenüber den Vergleichsverbindungen SdT1 und SdT2, bei denen kein bzw. ein über Stickstoff statt über den aromatischen Ring gebundener Carbazolsubstituent vorhanden ist (Beispiele V2, V5, E8). Insbesondere mit Verbindung 12f, bei der die Indenocarbazoleinheit über einen Phenylring mit der Triazineinheit verbunden ist, ergeben sich in diesem Fall sehr gute Leistungsdaten (Beispiel E2).

Zusammenfassend ergibt also die Kombination der Indenocarbazoleinheit mit der am aromatischen Ring gebundenen Carbazolgruppe hervorragende Leistungsdaten, die eine deutliche Verbesserung gegenüber Verbindungen darstellen, in denen keine Carbazolgruppe vorhanden ist, oder die Carbazolgruppe über den Stickstoff gebunden ist.

Die erfindungsgemäßen Materialien ergeben bei Einsatz als Matrixmaterialien in phosphoreszierenden OLEDs somit wesentliche Verbesserungen gegenüber dem Stand der Technik in allen Parametern, vor allem bezüglich Lebensdauer und Leistungseffizienz.

**Tabelle 1**

| Bsp. | HTL Dicke | IL Dicke | EBL Dicke | EML Dicke | HBL Dicke | ETL Dicke |
|---|---|---|---|---|---|---|
| V1 | SpA1 70nm | HATCN 5nm | SpMA1 90nm | SdT1:TEG1 (90%:10%) 30nm | SDT1 10nm | ST2:LiQ (50%:50%) 30nm |
| V2 | SpA1 70nm | HATCN 5nm | SpMA1 90nm | SdT1:TEG1 (95%:5%) 30nm | SDT1 10nm | ST2:LiQ (50%:50%) 30nm |
| V3 | SpA1 70nm | HATCN 5nm | SpMA1 90nm | SdT4:TEG1 (90%:10%) 30nm | SDT1 10nm | ST2:LiQ (50%:50%) 30nm |
| V4 | SpA1 70nm | HATCN 5nm | SpMA1 90nm | SdT2:TEG1 (90%:10%) 30nm | SDT1 10nm | ST2:LiQ (50%:50%) 30nm |
| V5 | SpA1 70nm | HATCN 5nm | SpMA1 90nm | SdT2:TEG1 (95%:5%) 30nm | SDT1 10nm | ST2:LiQ (50%:50%) 30nm |
| V6 | SpA1 70nm | HATCN 5nm | SpMA1 90nm | SdT3:TEG1 (90%:10%) 30nm | SDT1 10nm | ST2:LiQ (50%:50%) 30nm |
| V7 | SpA1 70nm | HATCN 5nm | SpMA1 90nm | SdT5:TEG1 (90%:10%) 30nm | SDT1 10nm | ST2:LiQ (50%:50%) 30nm |
| E1 | SpA1 70nm | HATCN 5nm | SpMA1 90nm | 12f:TEG1 (90%:10%) 30nm | SDT1 10nm | ST2:LiQ (50%:50%) 30nm |
| E2 | SpA1 70nm | HATCN 5nm | SpMA1 90nm | 12f:TEG1 (95%:5%) 30nm | SDT1 10nm | ST2:LiQ (50%:50%) 30nm |
| E3 | SpA1 70nm | HATCN 5nm | SpMA1 90nm | 12g:TEG1 (90%:10%) 30nm | SDT1 10nm | ST2:LiQ (50%:50%) 30nm |
| E4 | SpA1 70nm | HATCN 5nm | SpMA1 90nm | 12h:TEG1 (90%:10%) 30nm | SDT1 10nm | ST2:LiQ (50%:50%) 30nm |
| E5 | SpA1 70nm | HATCN 5nm | SpMA1 90nm | 12k:TEG1 (90%:10%) 30nm | SDT1 10nm | ST2:LiQ (50%:50%) 30nm |
| E6 | SpA1 70nm | HATCN 5nm | SpMA1 90nm | 12m:TEG1 (90%:10%) 30nm | SDT1 10nm | ST2:LiQ (50%:50%) 30nm |
| E7 | SpA1 70nm | HATCN 5nm | SpMA1 90nm | 12w:TEG1 (90%:10%) 30nm | SDT1 10nm | ST2:LiQ (50%:50%) 30nm |
| E8 | SpA1 70nm | HATCN 5nm | SpMA1 90nm | 12w:TEG1 (95%:5%) 30nm | SDT1 10nm | ST2:LiQ (50%:50%) 30nm |
| E9 | SpA1 90nm | HATCN 5nm | SpMA1 130nm | 12f:TER1 (94%:6%) 40nm | --- | ST2:LiQ (50%:50%) 40nm |

**Tabelle 2: Daten der OLEDs**

| Bsp. | U10 (V) | SE10 (cd/A) | LE10 (lm/W) | EQE10 | CIE x/ | L0;j0 | L1 % | LD (h) |
|---|---|---|---|---|---|---|---|---|
| V1 | 4.8 | 52 | 32 | 13.9% | 0.33/0.62 | 20mA/cm² | 80 | 145 |
| V2 | 4.7 | 46 | 31 | 12.4% | 0.34/0.63 | 20mA/cm² | 80 | 130 |
| V3 | 5.1 | 54 | 33 | 14.8% | 0.34/0.63 | 20mA/cm² | 80 | 120 |
| V4 | 4.5 | 44 | 31 | 12.1% | 0.33/0.63 | 20mA/cm² | 80 | 125 |
| V5 | 4.6 | 39 | 27 | 10.8% | 0.34/0.63 | 20mA/cm² | 80 | 110 |
| V6 | 5.5 | 47 | 27 | 13.0% | 0.34/0.63 | 20mA/cm² | 80 | 85 |
| V7 | 4.6 | 37 | 25 | 10.4% | 0.33/0.63 | 20mA/cm² | 80 | 120 |
| E1 | 4.2 | 54 | 40 | 14.6% | 0.33/0.62 | 20mA/cm² | 80 | 210 |
| E2 | 4.2 | 53 | 39 | 14.3% | 0.34/0.62 | 20mA/cm² | 80 | 185 |
| E3 | 4.4 | 48 | 34 | 13.3% | 0.33/0.63 | 20mA/cm² | 80 | 235 |
| E4 | 4.6 | 46 | 32 | 12.7% | 0.33/0.62 | 20mA/cm² | 80 | 195 |
| E5 | 4.7 | 41 | 27 | 11.3% | 0.32/0.62 | 20mA/cm² | 80 | 160 |
| E6 | 4.4 | 50 | 35 | 13.5% | 0.34/0.63 | 20mA/cm² | 80 | 220 |
| E7 | 4.7 | 49 | 33 | 13.8% | 0.33/0.62 | 20mA/cm² | 80 | 165 |
| E8 | 4.7 | 48 | 32 | 13.4% | 0.34/0.63 | 20mA/cm² | 80 | 180 |
| E9 | 4.8 | 12.7 | 8.4 | 13.9% | 0.67/0.33 | 4000 cd/m² | 80 | 470 |

**Tabelle 3: Strukturformeln der in den OLEDs verwendeten Materialien**

| | |
|---|---|
| 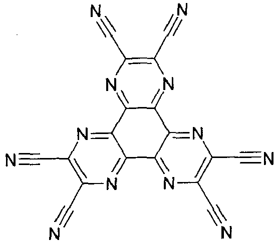 | 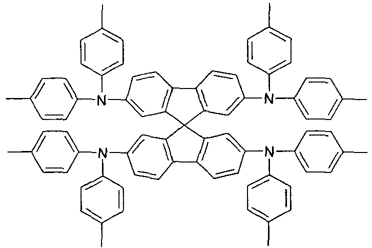 |
| HATCN | SpA1 |
| 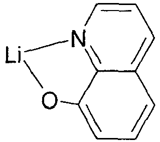 | 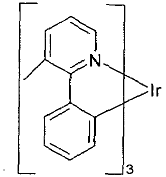 |
| LiQ | TEG1 |
| 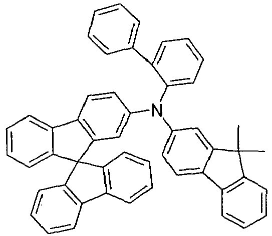 | 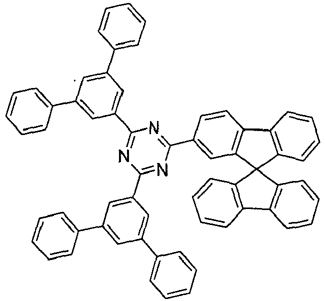 |
| SpMA1 | ST2 |
| 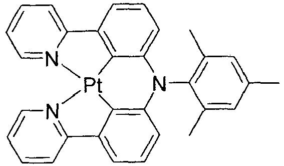 | 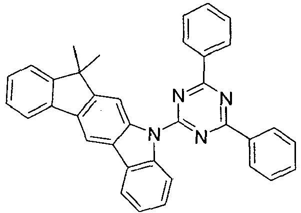 |
| TER1 | SdT1 |
| 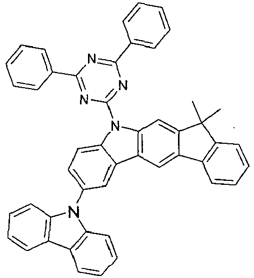 | 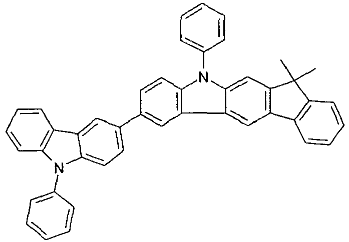 |
| SdT2 | SdT3 |
| 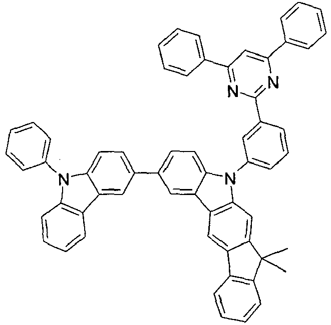 | 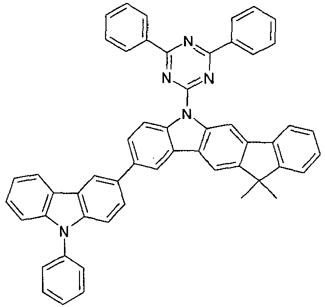 |
| SdT4 | SdT5 |
| 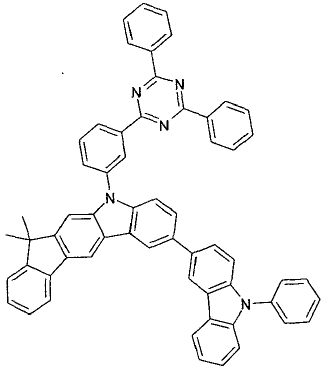 | 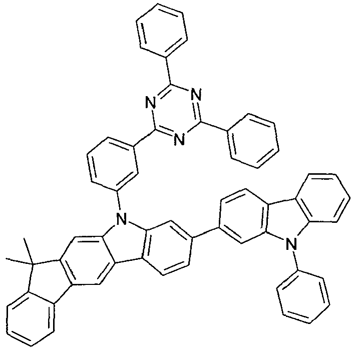 |
| 12f | 12g |
| 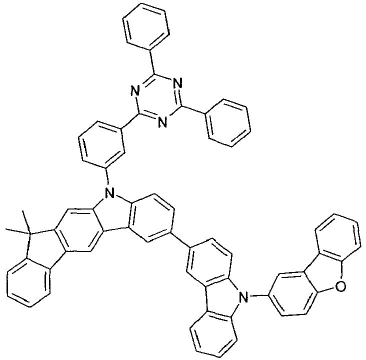 | 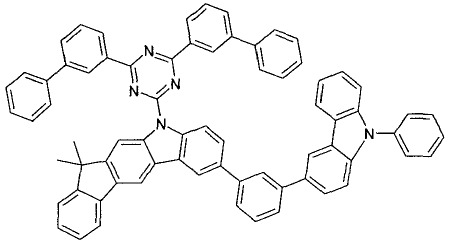 |
| 12h | 12k |
| 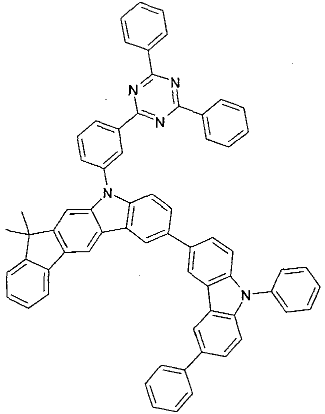 | 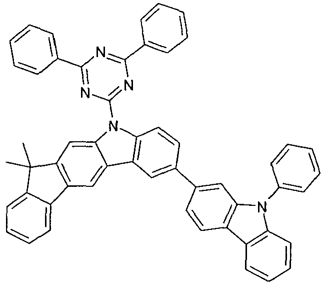 |
| 12m | 12w |

## Patentansprüche

1. Verbindung einer Formel (I) 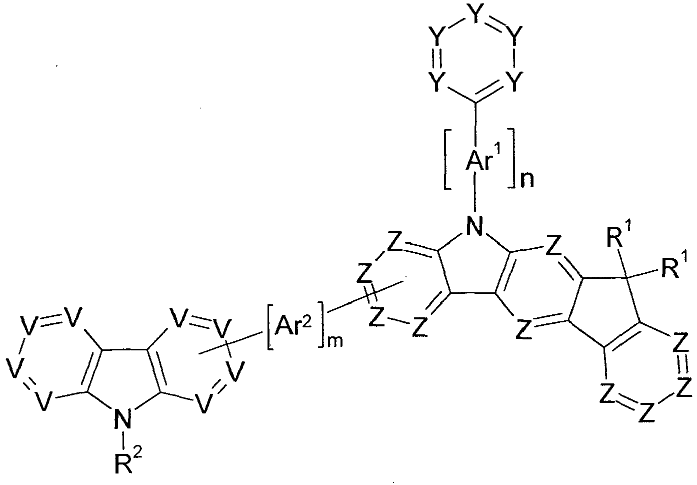 Formel (I), wobei gilt:
Y ist gleich N oder CR¹, wobei mindestens zwei Gruppen Y im Sechsring gleich N sein müssen;
Z ist gleich CR¹ oder N;
V ist gleich CR² oder N;
Ar¹ ist ein aromatisches oder heteroaromatisches Ringsystem mit 6 bis 40 aromatischen Ringatomen, das mit einem oder mehreren Resten R¹ substituiert sein kann;
Ar² ist ein aromatisches oder heteroaromatisches Ringsystem mit 6 bis 40 aromatischen Ringatomen, das mit einem oder mehreren Resten R¹ substituiert sein kann;
R¹ ist bei jedem Auftreten gleich oder verschieden H, D, F, Br, Cl, I, C(=O)R³, CN, Si(R³)₃, N(R³)₂, P(=O)(R³)₂, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch - R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann, wobei zwei oder mehr Reste R¹ miteinander verknüpft sein können und einen Ring bilden können;
R² ist bei jedem Auftreten gleich oder verschieden H, D, F, Br, Cl, I, C(=O)R³, CN, Si(R³)₃, N(R³)₂, P(=O)(R³)₂, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch - R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann;
R³ ist bei jedem Auftreten gleich oder verschieden H, D, F, Br, Cl, I, C(=O)R⁴, CN, Si(R⁴)₃, N(R⁴)₂, P(=O)(R⁴)₂, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch - R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxy- oder Heteroaryloxygruppe mit 5 bis 30 aromatischen Ringatomen, die durch einen oder mehrere Reste R⁴ substituiert sein kann, wobei zwei oder mehr Reste R³ miteinander verknüpft sein können und einen Ring bilden können;
R⁴ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden;
n ist gleich 0 oder 1;
m ist gleich 0 oder 1;
wobei der Index n gleich 0 ist, wenn in Formel (I) sowohl
a) nicht mehr als zwei Gruppen Y vorliegen, die gleich N sind, als auch
b) sich diese zwei Gruppen Y, die gleich N sind, in meta-Position zueinander am Sechsring befinden.

2. Verbindung nach Anspruch 1, **dadurch gekennzeichnet, dass** der Index m gleich 0 ist.

3. Verbindung nach Anspruch 1 oder 2, **dadurch gekennzeichnet, dass** der Index n gleich 0 oder 1 ist, wenn genau drei Gruppen Y im Sechsring gleich N sind, und der Index n in allen anderen Fällen gleich 0 ist.

4. Verbindung nach einem oder mehreren der Ansprüche 1 bis 3, **dadurch gekennzeichnet, dass** Z gleich CR¹ ist.

5. Verbindung nach einem oder mehreren der Ansprüche 1 bis 4, **dadurch gekennzeichnet, dass** V gleich CR² ist.

6. Verbindung nach einem oder mehreren der Ansprüche 1 bis 5, **dadurch gekennzeichnet, dass** genau drei Gruppen Y im Ring gleich N sind, und die restlichen Gruppen Y gleich CR¹ sind.

7. Verbindung nach einem oder mehreren der Ansprüche 1 bis 6, **dadurch gekennzeichnet, dass** Ar¹ eine Gruppe der folgenden Formel (Ar¹-I) darstellt 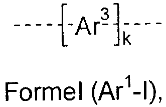 wobei die gestrichelten Linien die Bindungen an die Indenocarbazolgruppe und den Sechsring enthaltend die Gruppen Y darstellen,
Ar³ bei jedem Auftreten gleich oder verschieden eine Aryl- oder Heteroarylgruppe mit 6 bis 18 aromatischen Ringatomen ist, die mit einem oder mehreren Resten R¹ substituiert sein kann, wobei R¹ definiert ist wie in Anspruch 1; und
k 1, 2, 3 oder 4 ist, wobei der Index k so gewählt ist, dass die Zahl der aromatischen Ringatome der gesamten Gruppe Ar¹ die Zahl 40 nicht überschreitet.

8. Verbindung nach einem oder mehreren der Ansprüche 1 bis 7, **dadurch gekennzeichnet, dass** die Gruppe der Formel (Y) 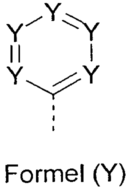 als Bestandteil der Formel (I) einer der folgenden Formeln (Y-1) bis (Y-6) entspricht, wobei die gestrichelte Linie die Bindung an den Rest der Verbindung bezeichnet,
| | |
|---|---|
| 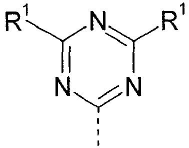 | 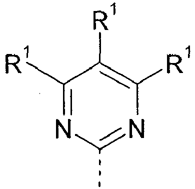 |
| Formel (Y-1) | Formel (Y-2) |
| 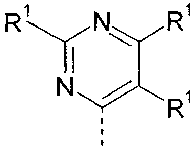 | 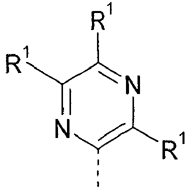 |
| Formel (Y-3) | Formel (Y-4) |
| 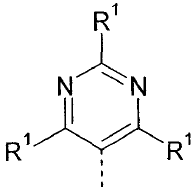 | 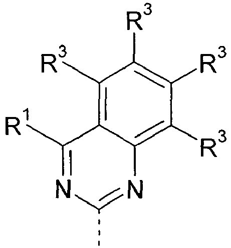 |
| Formel (Y-5) | Formel (Y-6) |
und wobei R¹ und R³ wie in Anspruch 1 definiert sind.

9. Oligomer, Polymer oder Dendrimer enthaltend eine oder mehrere Verbindungen nach einem oder mehreren der Ansprüche 1 bis 8, wobei die Bindung(en) zum Polymer, Oligomer oder Dendrimer an beliebigen, in Formel (I) mit R¹ oder R² substituierten Positionen lokalisiert sein können.

10. Formulierung, enthaltend mindestens eine Verbindung nach einem oder mehreren der Ansprüche 1 bis 8 oder mindestens ein Polymer, Oligomer oder Dendrimer nach Anspruch 9 sowie mindestens ein Lösungsmittel.

11. Elektronische Vorrichtung, gewählt aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs), **dadurch gekennzeichnet, dass** sie mindestens eine Verbindung nach einem oder mehreren der Ansprüche 1 bis 8 enthält.

12. Elektronische Vorrichtung nach Anspruch 11, gewählt aus organischen Elektrolumineszenzvorrichtungen, **dadurch gekennzeichnet, dass** sie Anode, Kathode und mindestens eine emittierende Schicht enthält, wobei mindestens eine Schicht der Vorrichtung, gewählt aus emittierenden Schichten, Elektronentransportschichten, Elektroneninjektionsschichten und Lochblockierschichten, mindestens eine Verbindung nach einem oder mehreren der Ansprüche 1 bis 8 enthält.

13. Elektronische Vorrichtung nach Anspruch 12, **dadurch gekennzeichnet, dass** die Verbindung nach einem oder mehreren der Ansprüche 1 bis 8 in einer emittierenden Schicht in Kombination mit einer oder mehreren phosphoreszierenden Emitterverbindungen vorhanden ist.

14. Verfahren zur Herstellung einer Verbindung nach einem oder mehreren der Ansprüche 1 bis 8, **dadurch gekennzeichnet, dass** eine Indenocarbazol-Verbindung mit einem aromatischen oder heteroaromatischen Ringsystem umgesetzt wird, welches eine elektronenarme Heteroarylgruppe enthält, wobei das aromatische oder heteroaromatische Ringsystem an das Stickstoffatom des Indenocarbazols gekuppelt wird.

15. Verwendung einer Verbindung nach einem oder mehreren der Ansprüche 1 bis 8 in einer elektronischen Vorrichtung.

## Claims

1. Compound of a formula (I) 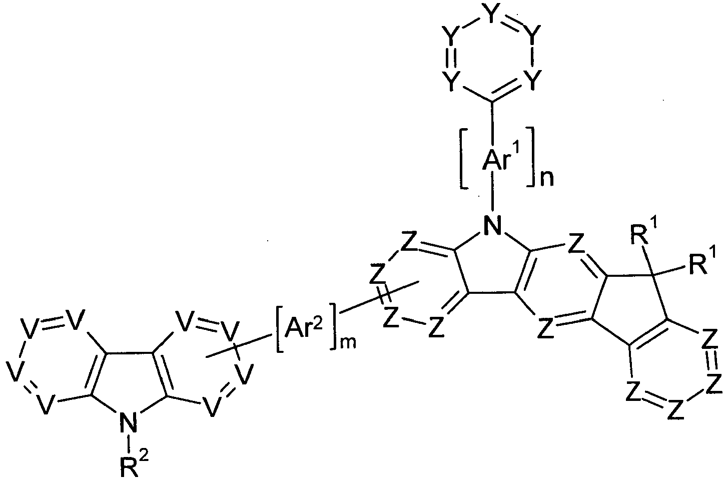 formula (I), where:
Y is equal to N or CR¹, where at least two groups Y in the six-membered ring must be equal to N;
Z is equal to CR¹ or N;
V is equal to CR² or N;
Ar¹ is an aromatic or heteroaromatic ring system having 6 to 40 aromatic ring atoms, which may be substituted by one or more radicals R¹;
Ar² is an aromatic or heteroaromatic ring system having 6 to 40 aromatic ring atoms, which may be substituted by one or more radicals R¹;
R¹ is on each occurrence, identically or differently, H, D, F, Br, Cl, I, C(=O)R³, CN, Si(R³)₃, N(R³)₂, P(=O)(R³)_{2,} S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C=C-, Si(R³)₂, C=O, C=NR³, -C(=O)O-, -C(=O)NR³-, NR³, P(=O)(R³), -O-, -S-, SO or SO₂, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³, where two or more radicals R¹ may be linked to one another and may form a ring;
R² is on each occurrence, identically or differently, H, D, F, Br, Cl, I, C(=O)R³, CN, Si(R³)₃, N(R³)₂, P(=O)(R³)₂, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C=C-, Si(R³)₂, C=O, C=NR³, -C(=O)O-, -C(=O)NR³-, NR³, P(=O)(R³), -O-, -S-, SO or SO₂, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³;
R³ is on each occurrence, identically or differently, H, D, F, Br, Cl, I, C(=O)R⁴, CN, Si(R⁴)₃, N(R⁴)₂, P(=O)(R⁴)₂, S(=O)R⁴, S(=O)₂R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R⁴ and where one or more CH₂ groups in the above-mentioned groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂, or an aromatic or heteroaromatic ring system having 5 to 30 aromatic ring atoms, which may in each case be substituted by one or more radicals R⁴, or an aryloxy or heteroaryloxy group having 5 to 30 aromatic ring atoms, which may be substituted by one or more radicals R⁴, where two or more radicals R³ may be linked to one another and may form a ring;
R⁴ is on each occurrence, identically or differently, H, D, F or an aliphatic, aromatic or heteroaromatic organic radical having 1 to 20 C atoms, in which, in addition, one or more H atoms may be replaced by D or F; two or more substituents R⁴ here may be linked to one another and may form a ring;
n is equal to 0 or 1;
m is equal to 0 or 1;
where the index n is equal to 0 if, in formula (I), both
a) not more than two groups Y which are equal to N are present and also
b) these two groups Y which are equal to N are located in the meta-position to one another on the six-membered ring.

2. Compound according to Claim 1, **characterised in that** the index m is equal to 0.

3. Compound according to Claim 1 or 2, **characterised in that** the index n is equal to 0 or 1 if precisely three groups Y in the six-membered ring are equal to N, and in all other cases the index n is equal to 0.

4. Compound according to one or more of Claims 1 to 3, **characterised in that** Z is equal to CR¹.

5. Compound according to one or more of Claims 1 to 4, **characterised in that** V is equal to CR².

6. Compound according to one or more of Claims 1 to 5, **characterised in that** precisely three groups Y in the ring are equal to N, and the remaining groups Y are equal to CR¹.

7. Compound according to one or more of Claims 1 to 6, **characterised in that** Ar¹ represents a group of the following formula (Ar¹-I) 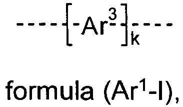 where the dashed lines represent the bonds to the indenocarbazole group and the six-membered ring containing the groups Y,
Ar³ is on each occurrence, identically or differently, an aryl or heteroaryl group having 6 to 18 aromatic ring atoms, which may be substituted by one or more radicals R¹, where R¹ is defined as in Claim 1; and
k is 1, 2, 3 or 4, where the index k is selected so that the number of aromatic ring atoms in the entire group Ar¹ does not exceed the number 40.

8. Compound according to one or more of Claims 1 to 7, **characterised in that** the group of the formula (Y) 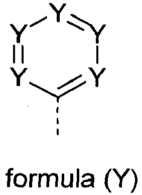 as constituent of the formula (I) conforms to one of the following formulae (Y-1) to (Y-6), where the dashed line denotes the bond to the remainder of the compound,
| | |
|---|---|
| 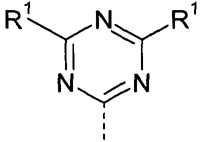 | 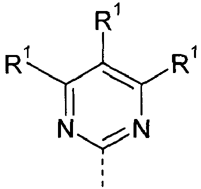 |
| formula (Y-1) | formula (Y-2) |
| 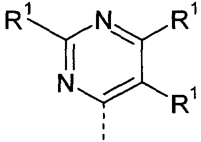 | 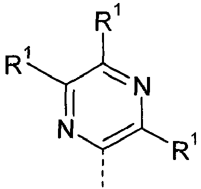 |
| formula (Y-3) | formula (Y-4) |
| 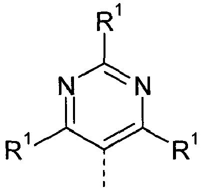 | 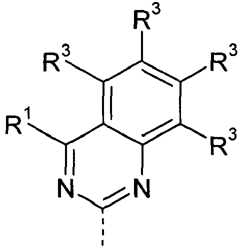 |
| formula (Y-5) | formula (Y-6) |
and where R¹ and R³ are as defined in Claim 1.

9. Oligomer, polymer or dendrimer containing one or more compounds according to one or more of Claims 1 to 8, where the bond(s) to the polymer, oligomer or dendrimer may be localised at any desired positions in formula (I) which are substituted by R¹ or R².

10. Formulation comprising at least one compound according to one or more of Claims 1 to 8 or at least one polymer, oligomer or dendrimer according to Claim 9 and at least one solvent.

11. Electronic device selected from organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs), **characterised in that** it comprises at least one compound according to one or more of Claims 1 to 8.

12. Electronic device according to Claim 11, selected from organic electroluminescent devices, **characterised in that** it comprises anode, cathode and at least one emitting layer, where at least one layer of the device, selected from emitting layers, electron-transport layers, electron-injection layers and hole-blocking layers, comprises at least one compound according to one or more of Claims 1 to 8.

13. Electronic device according to Claim 12, **characterised in that** the compound according to one or more of Claims 1 to 8 is present in an emitting layer in combination with one or more phosphorescent emitter compounds.

14. Process for the preparation of a compound according to one or more of Claims 1 to 8, **characterised in that** an indenocarbazole compound is reacted with an aromatic or heteroaromatic ring system which contains an electron-deficient heteroaryl group, where the aromatic or heteroaromatic ring system is coupled to the nitrogen atom of the indenocarbazole.

15. Use of a compound according to one or more of Claims 1 to 8 in an electronic device.

## Revendications

1. Composé de la formule (I) : 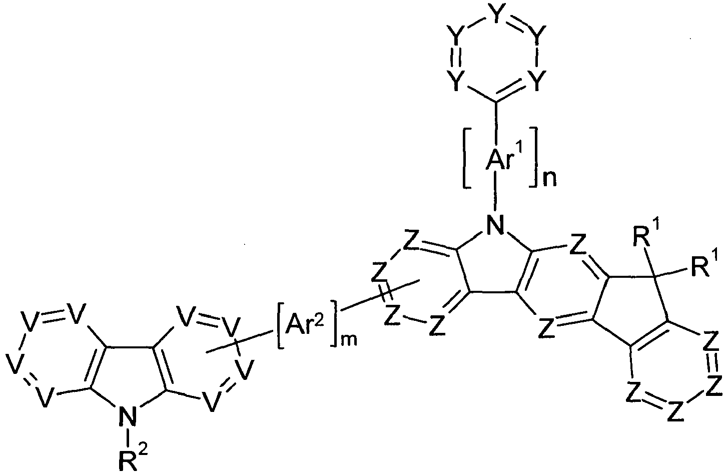 formule (I),
dans laquelle :
Y est égal à N ou CR¹, où au moins deux groupes Y dans le cycle à six éléments doivent être égaux à N ;
Z est égal à CR¹ ou N ;
V est égal à CR² ou N ;
Ar¹ est un système de cycle aromatique ou hétéroaromatique qui comporte 6 à 40 atomes de cycle aromatique, lequel peut être substitué par un radical ou plusieurs radicaux R¹ ;
Ar² est un système de cycle aromatique ou hétéroaromatique qui comporte 6 à 40 atomes de cycle aromatique, lequel peut être substitué par un radical ou plusieurs radicaux R¹ ;
R¹ est, pour chaque occurrence, de manière identique ou différente, H, D, F, Br, Cl, I, C(=O)R³, CN, Si(R³)₃, N(R³)₂, P(=O)(R³)₂, S(=O)R³, S(=O)₂R³, un groupe alkyle ou alcoxy en chaîne droite qui comporte 1 à 20 atome(s) de C ou un groupe alkyle ou alcoxy ramifié ou cyclique qui comporte 3 à 20 atomes de C ou un groupe alkényle ou alkynyle qui comporte 2 à 20 atomes de C, où les groupes qui ont été mentionnés ci avant peuvent chacun être substitués par un radical ou plusieurs radicaux R³ et où un ou plusieurs groupe(s) CH₂ dans les groupes qui ont été mentionnés ci avant peut/peuvent être remplacé(s) par -R³C=CR³-, -C≡C-, Si(R³)₂, C=O, C=NR³, -C(=O)O-, -C(=O)NR³-, NR³, P(=O)(R³), -O-, -S-, SO ou SO₂, ou un système de cycle aromatique ou hétéroaromatique qui comporte 5 à 30 atomes de cycle aromatique, lequel peut, dans chaque cas, être substitué par un radical ou plusieurs radicaux R³, ou un groupe aryloxy ou hétéroaryloxy qui comporte 5 à 30 atomes de cycle aromatique, lequel peut être substitué par un radical ou plusieurs radicaux R³, où deux radicaux R¹ ou plus peuvent être liés l'un à l'autre ou les uns aux autres et peuvent former un cycle ;
R² est, pour chaque occurrence, de manière identique ou différente, H, D, F, Br, Cl, I, C(=O)R³, CN, Si(R³)₃, N(R³)₂, P(=O)(R³)₂, S(=O)R³, S(=O)₂R³, un groupe alkyle ou alcoxy en chaîne droite qui comporte 1 à 20 atome(s) de C ou un groupe alkyle ou alcoxy ramifié ou cyclique qui comporte 3 à 20 atomes de C ou un groupe alkényle ou alkynyle qui comporte 2 à 20 atomes de C, où les groupe qui ont été mentionnés ci avant peuvent chacun être substitués par un radical ou plusieurs radicaux R³ et où un ou plusieurs groupe(s) CH₂ dans les groupes qui ont été mentionnés ci avant peut/peuvent être remplacé(s) par -R³C=CR³-, -C≡C-, Si(R³)₂, C=O, C=NR³, -C(=O)O-, -C(=O)NR³-, NR³, P(=O)(R³), -O-, -S-, SO ou SO₂, ou un système de cycle aromatique ou hétéroaromatique qui comporte 5 à 30 atomes de cycle aromatique, lequel peut, dans chaque cas, être substitué par un radical ou plusieurs radicaux R³, ou un groupe aryloxy ou hétéroaryloxy qui comporte 5 à 30 atomes de cycle aromatique, lequel peut être substitué par un radical ou plusieurs radicaux R³ ;
R³ est, pour chaque occurrence, de manière identique ou différente, H, D, F, Br, Cl, I, C(=O)R⁴, CN, Si(R⁴)₃, N(R⁴)₂, P(=O)(R⁴)_{2,} S(=O)R⁴, S(=O)₂R⁴, un groupe alkyle ou alcoxy en chaîne droite qui comporte 1 à 20 atome(s) de C ou un groupe alkyle ou alcoxy ramifié ou cyclique qui comporte 3 à 20 atomes de C ou un groupe alkényle ou alkynyle qui comporte 2 à 20 atomes de C, où les groupes qui ont été mentionnés ci avant peuvent chacun être substitués par un radical ou plusieurs radicaux R⁴ et où un ou plusieurs groupe(s) CH₂ dans les groupes qui ont été mentionnés ci avant peut/peuvent être remplacé(s) par -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO ou SO₂, ou un système de cycle aromatique ou hétéroaromatique qui comporte 5 à 30 atomes de cycle aromatique, lequel peut, dans chaque cas, être substitué par un radical ou plusieurs radicaux R⁴, ou un groupe aryloxy ou hétéroaryloxy qui comporte 5 à 30 atomes de cycle aromatique, lequel peut être substitué par un radical ou plusieurs radicaux R⁴, où deux radicaux R³ ou plus peuvent être liés l'un à l'autre ou les uns aux autres et peuvent former un cycle ;
R⁴ et, pour chaque occurrence, de manière identique ou différente, H, D, F ou un radical organique aliphatique, aromatique ou hétéroaromatique qui comporte 1 à 20 atome(s) de C, où, en outre, un ou plusieurs atome(s) de H peut/peuvent être remplacé(s) par D ou F ; deux substituants R⁴ ou plus peuvent ici être liés l'un à l'autre ou les uns aux autres et peuvent former un cycle ;
n est égal à 0 ou 1 ;
m est égal à 0 ou 1 ;
où l'indice n est égal à 0 si, dans la formule (I), à la fois :
a) pas plus de deux groupes Y qui sont égaux à N sont présents et également
b) ces deux groupes Y qui sont égaux à N sont situés à la position méta, de façon mutuelle, du cycle à six éléments.

2. Composé selon la revendication 1, **caractérisé en ce que** l'indice m est égal à 0.

3. Composé selon la revendication 1 ou 2, **caractérisé en ce que** l'indice n est égal à 0 ou 1 si précisément trois groupes Y dans le cycle à six éléments sont égaux à N, et dans tous les autres cas, l'indice n est égal à 0.

4. Composé selon une ou plusieurs des revendications 1 à 3, **caractérisé en ce que** Z est égal à CR¹.

5. Composé selon une ou plusieurs des revendications 1 à 4, **caractérisé en ce que** V est égal à CR².

6. Composé selon une ou plusieurs des revendications 1 à 5, **caractérisé en ce que** précisément trois groupes Y dans le cycle sont égaux à N, et les groupes Y restants sont égaux à CR¹.

7. Composé selon une ou plusieurs des revendications 1 à 6, **caractérisé en ce que** Ar¹ représente un groupe de la formule (Ar¹-I) qui suit : 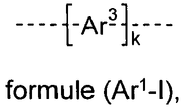 dans laquelle les lignes en pointillés représentent les liaisons sur le groupe indénocarbazole et le cycle à six éléments contenant les groupes Y,
Ar³ est, pour chaque occurrence, de manière identique ou différente, un groupe aryle ou hétéroaryle qui comporte 6 à 18 atomes de cycle aromatique, lequel peut être substitué par un radical ou plusieurs radicaux R¹, où R¹ est défini comme selon la revendication 1 ; et
k est 1, 2, 3 ou 4, où l'indice k est sélectionné de telle sorte que le nombre d'atomes de cycle aromatique dans le groupe Ar¹ complet n'excède pas le nombre 40.

8. Composé selon une ou plusieurs des revendications 1 à 7, **caractérisé en ce que** le groupe de la formule (Y) : 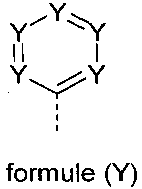 en tant que constituant de la formule (I) est conforme à l'une des formules (Y-1) à (Y-6) qui suivent, dans lesquelles la ligne en pointillés représente la liaison sur le reste du composé :
| | |
|---|---|
| 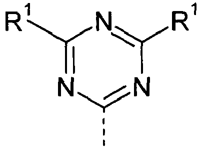 | 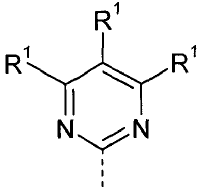 |
| formule (Y-1) | formule (Y-2) |
| 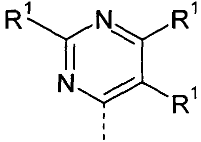 | 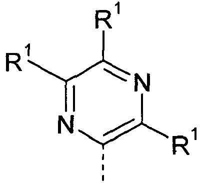 |
| formule (Y-3) | formule (Y-4) |
| 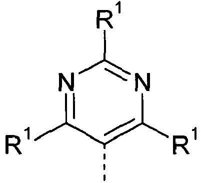 | 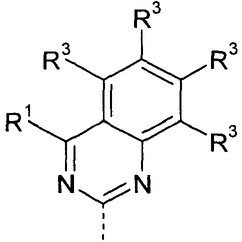 |
| formule (Y-5) | formule (Y-6) |
et dans lesquelles R¹ et R³ sont comme défini selon la revendication 1.

9. Oligomère, polymère ou dendrimère contenant un ou plusieurs composé(s) selon une ou plusieurs des revendications 1 à 8, où la/les liaison(s) sur le polymère, l'oligomère ou le dendrimère peut/peuvent être localisée(s) au niveau de n'importe quelles positions souhaitées dans la formule (I) qui sont substituées par R¹ ou par R².

10. Formulation comprenant au moins un composé selon une ou plusieurs des revendications 1 à 8 ou au moins un polymère, un oligomère ou un dendrimère selon la revendication 9 et au moins un solvant.

11. Dispositif électronique sélectionné parmi les circuits intégrés organiques (OIC), les transistors à effet de champ organiques (OFET), les transistors à film mince organiques (OTFT), les transistors à émission de lumière organiques (OLET), les cellules solaires organiques (OSC), les détecteurs optiques organiques, les photorécepteurs organiques, les dispositifs à extinction de champ organiques (OFQD), les cellules électrochimiques à émission de lumière organiques (OLEC), les diodes laser organiques (O-laser) et les dispositifs électroluminescents organiques (OLED), **caractérisé en ce qu'**il comprend au moins un composé selon une ou plusieurs des revendications 1 à 8.

12. Dispositif électronique selon la revendication 11, sélectionné parmi les dispositifs électroluminescents organiques, **caractérisé en ce qu'**il comprend une anode, une cathode et au moins une couche d'émission, où au moins une couche du dispositif, qui est sélectionnée parmi les couches d'émission, les couches de transport d'électrons, les couches d'injection d'électrons et les couches de blocage de trous, comprend au moins un composé selon une ou plusieurs des revendications 1 à 8.

13. Dispositif électronique selon la revendication 12, **caractérisé en ce que** le composé selon une ou plusieurs des revendications 1 à 8 est présent dans une couche d'émission en combinaison avec un ou plusieurs composé(s) émetteur(s) de phosphorescence.

14. Procédé pour la préparation d'un composé selon une ou plusieurs des revendications 1 à 8, **caractérisé en ce qu'**un composé indénocarbazole est amené à réagir avec un système de cycle aromatique ou hétéroaromatique qui contient un groupe hétéroaryle déficient en électrons, où le système de cycle aromatique ou hétéroaromatique est couplé à l'atome d'azote de l'indénocarbazole.

15. Utilisation d'un composé selon une ou plusieurs des revendications 1 à 8 dans un dispositif électronique.
